# EUROPEAN PATENT APPLICATION

(11) **EP 3 660 702 A1**
(43) Date of publication of application: **03.06.2020**
(21) Application number: 18838660.1
(22) Date of filing: 11.06.2018
(51) Int. Cl.: G06F 16/9035

(54) **INFORMATION PROCESSING DEVICE, INFORMATION PROCESSING METHOD, AND PROGRAM**

(30) Priority: 28.07.2017 JP 2017146012
(71) Applicant: Sony Corporation, Tokyo 108-0075 (JP)
(72) Inventor: AIZAWA, Kota, Tokyo 108-0075 (JP); WAKITA, Yoshihiro, Tokyo 108-0075 (JP)
(74) Representative: MFG Patentanwälte Meyer-Wildhagen Meggle-Freund Gerhard PartG mbB
(86) International application number: PCT/JP2018/022165
(87) International publication number: WO 2019/021653

(57) **Abstract**

[Problem] To provide an information processing device, an information processing method, and a computer program that are capable of dealing with needs potentially generated in an estimated user state. [Solution] Provided is an information processing device including: an estimation unit configured to estimate states of one or a plurality of target users based on sensing data; and an output control unit configured to perform output corresponding to an estimation result.

## Description

### Field

The present disclosure relates to an information processing device, an information processing method, and a computer program.

### Background

A recently developed technology detects sensing data such as biological information of a user and provides information related to the state of the user estimated based on the detected sensing data. For example, Patent Literature 1 below discloses an information processing device configured to estimate the states of a plurality of users based on sensing data obtained from the users and generate information indicating the estimated states of the users.

### Citation List

### Patent Literature

Patent Literature 1: JP 2014-134922 A

### Summary

### Technical Problem

In the above-described technology disclosed in Patent Literature 1, basically, the state of each user is estimated based on the sensing data, and a result of the estimation is simply output toward the user, and needs of the user that could occur in the estimated state are not dealt with.

The present disclosure discloses an information processing device, an information processing method, and a computer program that are novel and modified and can deal with needs that could occur in an estimated user state.

### Solution to Problem

According to the present disclosure, an information processing device is provided that includes: an estimation unit configured to estimate states of one or a plurality of target users based on sensing data; and an output control unit configured to perform output corresponding to an estimation result.

According to the present disclosure, an information processing method is provided that includes: estimating states of one or a plurality of target users based on sensing data; and performing output corresponding to an estimation result.

According to the present disclosure, a computer program is provided that configured to cause a computer to execute: a function to estimate states of one or a plurality of target users based on sensing data; and a function to perform output corresponding to an estimation result.

### Advantageous Effects of Invention

As described above, according to the present disclosure, it is possible to provide an information processing device, an information processing method, and a computer program that are capable of dealing with needs potentially generated in an estimated user state.

The above-described effect is not necessarily restrictive, and any effect indicated in the present specification or any other effect that could be understood from the present specification may be achieved together with or in place of the above-described effect.

### Brief Description of Drawings

FIG. 1 is an explanatory diagram for describing an exemplary configuration of an information processing system 1 according to an embodiment of the present disclosure.
FIG. 2 is a block diagram illustrating an exemplary configuration of a wearable device 10 according to a first embodiment.
FIG. 3 is an explanatory diagram for describing a PPG sensor unit 122 according to the embodiment.
FIG. 4 is an explanatory diagram illustrating an exemplary pulse wave signal that can be obtained by the PPG sensor unit 122 according to the embodiment.
FIG. 5 is an explanatory diagram illustrating exemplary appearance of the wearable device 10 according to the embodiment.
FIG. 6 is a block diagram illustrating an exemplary configuration of a server 30 according to the embodiment.
FIG. 7 is an explanatory diagram (1) illustrating an exemplary HRV index that can be obtained by an estimation unit 340 according to the embodiment.
FIG. 8 is an explanatory diagram (2) illustrating the exemplary HRV index that can be obtained by the estimation unit 340 according to the embodiment.
FIG. 9 is an explanatory diagram (1) for describing exemplary machine learning by a learning unit 342 according to the embodiment.
FIG. 10 is an explanatory diagram (2) for describing the exemplary machine learning by the learning unit 342 according to the embodiment.
FIG. 11 is a block diagram illustrating an exemplary configuration of a terminal 50 according to the embodiment.
FIG. 12 is a flowchart for describing an exemplary information processing method according to the embodiment.
FIG. 13 is an explanatory diagram illustrating an exemplary setting screen 800 of the terminal 50 according to the embodiment.
FIG. 14 is an explanatory diagram for describing Modification 1 of the embodiment.
FIG. 15 is a flowchart for describing an exemplary information processing method according to Modification 2 of the embodiment.
FIG. 16 is an explanatory diagram illustrating an exemplary display lamp 700 according to Modification 2 of the embodiment.
FIG. 17 is an explanatory diagram illustrating an exemplary display screen 802 according to Modification 2 of the embodiment.
FIG. 18 is an explanatory diagram illustrating an exemplary display screen 804 according to Modification 3 of the embodiment.
FIG. 19 is an explanatory diagram illustrating an exemplary display screen 806 according to Modification 3 of the embodiment.
FIG. 20 is a flowchart for describing an exemplary information processing method according to a second embodiment of the present disclosure.
FIG. 21 is an explanatory diagram (1) for describing exemplary information processing according to the embodiment.
FIG. 22 is an explanatory diagram (2) for describing the exemplary information processing according to the embodiment.
FIG. 23 is an explanatory diagram (1) for describing exemplary information processing according to Modification 1 of the embodiment.
FIG. 24 is an explanatory diagram (2) for describing the exemplary information processing according to Modification 1 of the embodiment.
FIG. 25 is an explanatory diagram (1) for describing exemplary information processing according to Modification 3 of the embodiment.
FIG. 26 is an explanatory diagram (2) for describing the exemplary information processing according to Modification 3 of the embodiment.
FIG. 27 is a flowchart for describing an exemplary information processing method according to a third embodiment of the present disclosure.
FIG. 28 is an explanatory diagram for describing the exemplary information processing according to the embodiment.
FIG. 29 is a block diagram illustrating an exemplary hardware configuration of an information processing device 900 according to an embodiment of the present disclosure.

### Description of Embodiments

Preferable embodiments of the present disclosure will be described below in detail with reference to the accompanying drawings. In the present specification and drawings, components having functional configurations identical to each other in effect are denoted by an identical reference sign, and duplication description thereof will be omitted.

The description is performed in the following order.
1. Overview of information processing system 1 according to embodiment of the present disclosure
2. Technological background of creation of embodiments according to the present disclosure
3. First embodiment
   3.1 Detailed configuration of wearable device 10
   3.2 Detailed configuration of server 30
   3.3 Detailed configuration of control unit 330
   3.4 Detailed configuration of terminal 50
   3.5 Information processing method
   3.6 Modification 1
   3.7 Modification 2
   3.8 Modification 3
4. Second embodiment
   4.1 Information processing method
   4.2 Modification 1
   4.3 Modification 2
   4.4 Modification 3
   4.5 Modification 4
5. Third embodiment
   5.1 Information processing method
   5.2 Modification 1
   5.3 Modification 2
6. Summary
7. Hardware configuration
8. Supplement

<<1. Overview of information processing system 1 according to embodiment of the present disclosure>>

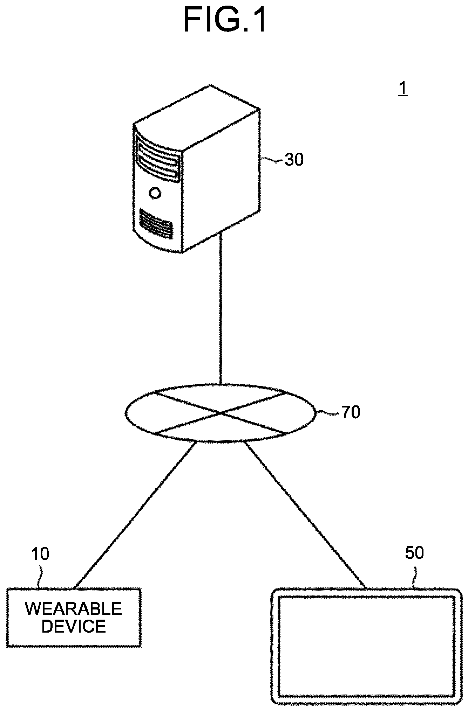

The following first schematically describes an information processing system 1 according to an embodiment of the present disclosure with reference to FIG. 1. FIG. 1 is an explanatory diagram for describing an exemplary configuration of the information processing system 1 according to the present embodiment.

As illustrated in FIG. 1, the information processing system 1 according to the present embodiment includes a wearable device 10, a server 30, and a terminal 50 connected with each other to perform communication therebetween through a network 70. Specifically, the wearable device 10, the server 30, and the terminal 50 are connected with the network 70 through a base station (not illustrated) (for example, a cellular phone base station or a wireless LAN access point). A communication scheme employed for the network 70 may be an optional wired or wireless (for example, WiFi (registered trademark) or Bluetooth (registered trademark)) scheme, but is desirably a communication scheme that allows maintenance of stable operation.

### (Wearable device 10)

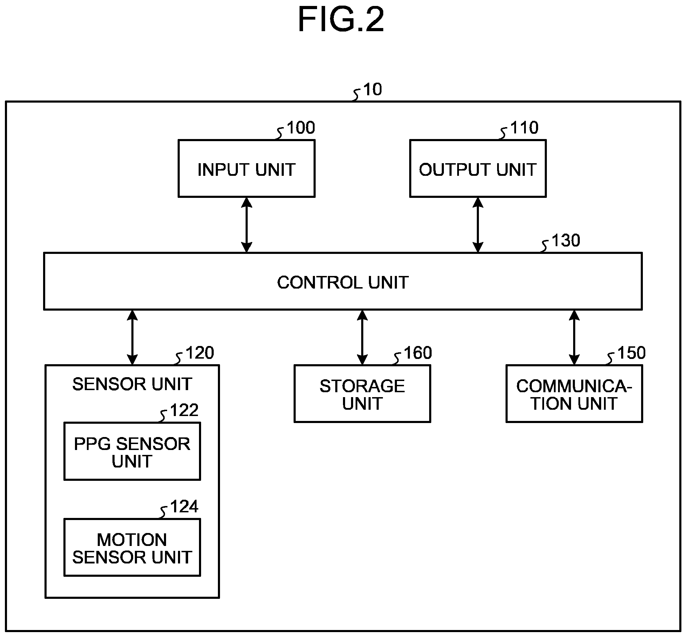

The wearable device 10 may be a device attachable to part (for example, ear lobe, neck, arm, wrist, or ankle) of the body of a user or an implant device (implant terminal) inserted in the body of the user. More specifically, the wearable device 10 may be a wearable device of various schemes such as a head mounted display (HMD) type, an eye glasses type, an ear device type, an anklet type, an armlet (wrist band) type, a collar type, an eyewear type, a pad type, a badge type, and a cloth type. In addition, the wearable device 10 includes a sensor unit 120 including a sensor such as a photo plethysmography (PPG) sensor unit 122 configured to detect a pulse wave signal of the pulse rate of the user (refer to FIG. 2). In the present embodiment, for example, the physical and mental states (in the following description, the physical and mental states of the user are collectively referred to as "emotion"), the motion state, and the current position of the user can be estimated based on sensing data acquired by the sensor unit 120. In addition, in the present embodiment, for example, the consumption energy, the number of steps, the amount of motion, and the state of sleep of the user may be estimated based on the sensing data acquired by the sensor unit 120 included in the wearable device 10. In the following description, the wearable device 10 is assumed to be a wearable device of an armlet (wristband) type. Details of the wearable device 10 will be described later.

### (Server 30)

The server 30 is achieved by, for example, a computer. For example, the server 30 processes the sensing data acquired by the wearable device 10, and outputs information obtained by this processing to another device (for example, the terminal 50). Details of the server 30 will be described later.

### (Terminal 50)

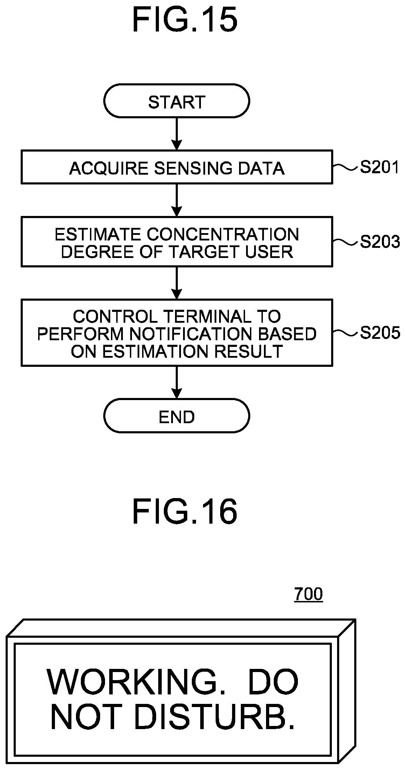

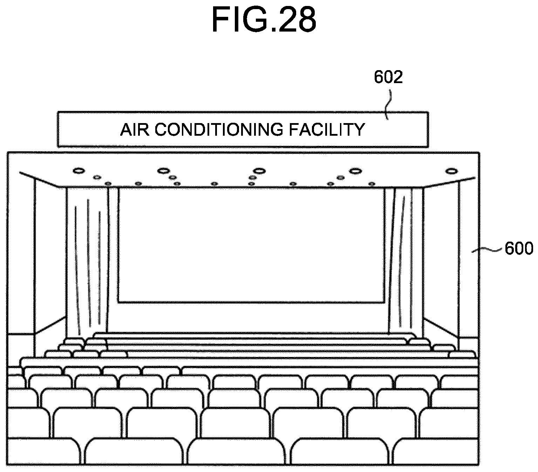

The terminal 50 is a terminal used by the user or installed near the user and configured to output the information obtained by the server 30 to the user. The terminal 50 may receive information input by the user and may output the received information as sensing data to the server 30. For example, the terminal 50 may be a device such as a tablet personal computer (PC), a smartphone, a cellular phone, a laptop PC, a notebook PC, or a HMD. In addition, the terminal 50 includes, for example, a display unit (not illustrated) configured to perform display to the user, an operation unit (not illustrated) configured to receive an operation from the user, a speaker (not illustrated) configured to perform voice output to the user, and a microphone (not illustrated) configured to acquire voice from surroundings. In the present embodiment, the terminal 50 is not limited to a PC as described above but may be a device that can output information to the user and can be controlled for the user, and may be, for example, a display lamp 700 (refer to FIG. 16) or an air conditioning facility 602 (refer to FIG. 28).

In the present embodiment, the sensor unit 120 included in the wearable device 10 may be provided to the terminal 50 or may be provided separately from the wearable device 10, the terminal 50, and the like.

In FIG. 1, the information processing system 1 according to the present embodiment includes one wearable device 10 and one terminal 50, but in the present embodiment, is not limited thereto. For example, the information processing system 1 according to the present embodiment may include a plurality of wearable devices 10 and a plurality of terminals 50. In addition, the information processing system 1 according to the present embodiment may include, for example, another communication device such as a relay device used to transmit information from the wearable device 10 to the server 30. Alternatively, the information processing system 1 according to the present embodiment may include no wearable device 10, and in such a case, for example, the terminal 50 functions as the wearable device 10, and sensing data acquired by the terminal 50 is output to the server 30. In the following description, sensing data not only means data from a measurement device configured to measure a particular phenomenon by detecting, for example, an electromagnetic, optical, mechanical, or thermal characteristic, but also includes context information (information such as schedule, sex, and age) related to the user.

### <<2. Technological background of creation of embodiments according to the present disclosure>>

The above description is an overview of the information processing system 1 according to the embodiment of the present disclosure, and the wearable device 10, the server 30, and the terminal 50 included in the information processing system 1. Subsequently, the technological background for the inventors to create embodiments according to the present disclosure will be described below before description of details of each embodiment according to the present disclosure.

There has been an information processing system configured to estimate, for example, the emotion of the user based on various kinds of sensing data acquired by a sensor unit provided to a wearable device mounted on the user. For example, when the sensor unit is a sensor configured to detect the pulse rate of the user, the information processing system estimates, for example, the motion amount of the user based on the detected pulse rate and notifies a result of the estimation to the user. Then, the user refers to the notified estimation result to manage the physical condition of the user or the like. In this manner, the information processing system only estimates the state of the user based on the sensing data and outputs a result of the estimation to the user.

When the information processing system is used in a group of a plurality of users at a workplace or the like, the information processing system only estimates the state of each user belonging to the workplace and notifies a result of the estimation to the user or another user at the workplace.

In such a situation, while understanding the convenience of the above-described information processing system, the inventors conducted intensive studies to further improve the convenience of the information processing system. Through such studies, the inventors have found that the convenience of the information processing system can be improved by not only estimating the state of a user but also dealing with needs of the user that could occur in the estimated state.

Accordingly, the inventors have reached creation of embodiments of the present disclosure from the above-described finding as one viewpoint. Specifically, according to the embodiments of the present disclosure describes below, it is possible to provide the information processing system 1 capable of dealing with needs that could occur in an estimated user state. Details of such embodiments of the present disclosure will be sequentially described below.

In the following description, a user means a general user who uses the information processing system 1 according to the embodiment of the present disclosure, and a target user means a target, the state or the like of which is sensed by, for example, the sensor unit 120 of the wearable device 10 among the users. The user includes the target user.

### <<3. First embodiment>>

The following first describes a first embodiment according to the present disclosure. In the present embodiment, the information processing system 1 estimates the state of a target user based on sensing data and controls, for example, the terminal 50 used by the user in accordance with a result of the estimation. More specifically, in the present embodiment, the information processing system 1 estimates emotion (concentration degree) of the target user based on sensing data obtained by the sensor unit 120 of the wearable device 10 mounted on the target user, and controls a smartphone (the terminal 50) used by the target user in accordance with a result of the estimation.

More specifically, assume that the target user has started work in an office and is highly concentrated. In such a case, the target user often feels unpleasant about the sound of an alarm of a smartphone (the terminal 50) used by the target user upon an incoming alert because the sound may interrupt the work. Thus, in the present embodiment, when it is estimated that the concentration degree of the target user is high, the smartphone of the target user is controlled not to sound an alarm upon an incoming alert. As a result, the target user can continue concentrating on his work, which leads to improved performance of the target user. In other words, in the present embodiment, the terminal 50 or the like is controlled not only to estimate the emotion of the target user but also specify needs of the target user in accordance with the estimated emotion and deal with the specified needs. Thus, according to the present embodiment, the target user can create a situation preferable for the target user without taking any action in particular. In the following description, the state of a high concentration degree means a mental state in which a person is focused on a single matter while working.

The following first describes details of each device included in the information processing system 1 according to the present embodiment. Specifically, as described above, the information processing system 1 according to the present embodiment includes the wearable device 10, the server 30, and the terminal 50.

### <3.1 Detailed configuration of wearable device 10>

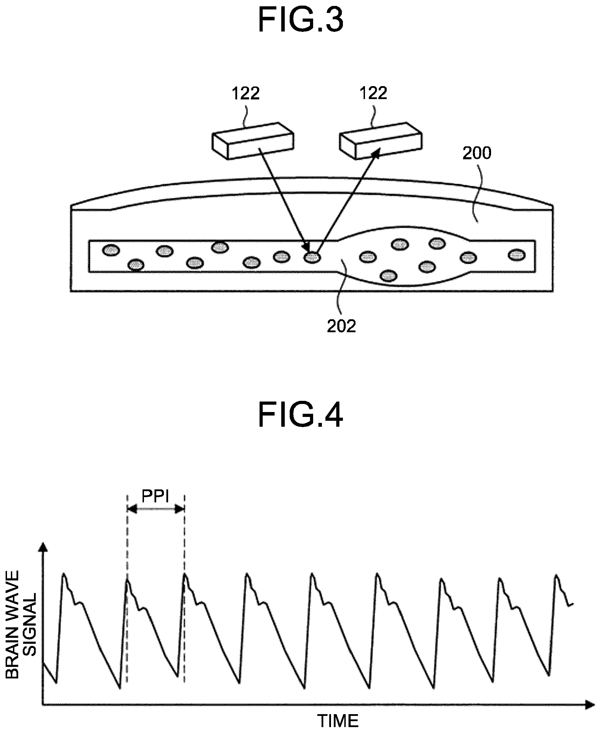

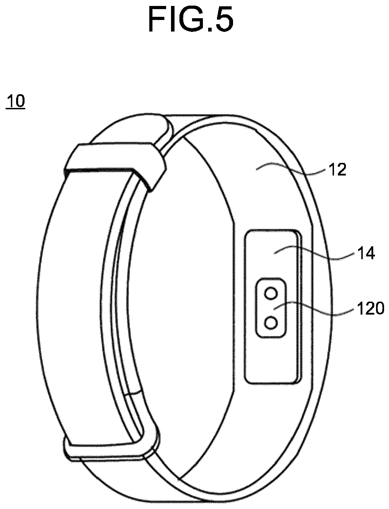

First, the following description will be made on a detailed configuration of the wearable device 10 according to the embodiment of the present disclosure with reference to FIGS. 2 to 5. FIG. 2 is a block diagram illustrating an exemplary configuration of the wearable device 10 according to the present embodiment, and FIG. 3 is an explanatory diagram for describing a PPG sensor unit 122 according to the present embodiment. FIG. 4 is an explanatory diagram illustrating an exemplary pulse wave signal that can be obtained by the PPG sensor unit 122 according to the present embodiment, and FIG. 5 is an explanatory diagram illustrating exemplary appearance of the wearable device 10 according to the present embodiment.

As illustrated in FIG. 2, the wearable device 10 mainly includes an input unit 100, an output unit 110, the sensor unit 120, a control unit 130, a communication unit 150, and a storage unit 160. Details of each functional component of the wearable device 10 will be described below.

### (Input unit 100)

The input unit 100 receives inputting of data and commands from the target user to the wearable device 10. More specifically, the input unit 100 is achieved by, for example, a touch panel, a button, or a microphone.

### (Output unit 110)

The output unit 110 is a device for presenting information to the target user, and for example, outputs various kinds of information to the target user by image, voice, light, vibration, or the like. More specifically, the output unit 110 can notify reception of a mail or the like to the target user and display, on a screen, information provided by the server 30 to be described later. The output unit 110 is achieved by, for example, a display, a speaker, an earphone, a light emitting element (for example, a light emitting diode (LED)), or a vibration module. Part of the function of the output unit 110 may be provided by the terminal 50.

### (Sensor unit 120)

The sensor unit 120 includes various sensors that are provided in the wearable device 10 mounted on the body of the target user and detect the state of the target user. Specifically, the sensor unit 120 includes the PPG sensor unit (beat sensor) 122 configured to detect the pulse rate or heart rate of the target user and acquire temporally sequential data (pulse wave signal) of the heart rate or pulse rate, a motion sensor unit 124 configured to detect motion of the target user, and the like.

### - PPG sensor unit 122 -

The PPG sensor unit 122 is a biosensor mounted on a body part (for example, arm, wrist, or ankle) such as skin of the target user to detect a pulse wave signal of the target user. The pulse wave signal is a waveform generated by beat of an artery appearing on the body surface or the like upon pressure change caused on the artery inner wall by blood transfer to the whole body through arteries due to contraction (beat; the number of beats at the heart in unit time is referred to as a heart rate) of muscle of the heart in constant rhythm. As illustrated in FIG. 3, to acquire the pulse wave signal, the PPG sensor unit 122 irradiates, with light, a blood vessel 202 in a measurement site 200 of the target user, such as a hand, an arm, or a leg and detects light scattered by a material moving in the blood vessel of the target user or a biological tissue at rest. The amount of absorbed light is proportional to the amount of blood flowing through the blood vessel 202 in the measurement site 200 because irradiation light is absorbed by red blood cells in the blood vessel 202. Thus, the PPG sensor unit 122 can sense a change in the amount of flowing blood by detecting the intensity of the scattered light. In addition, the waveform of beat, in other words, the pulse wave signal as illustrated in FIG. 4 can be detected based on this change in the blood current amount. Such a method is called a photo plethysmography (PPG) method.

Specifically, the PPG sensor unit 122 includes, for example, a small-sized laser or light emitting diode (LED) (not illustrated) capable of emitting coherent light, and emits light having a predetermined wavelength such as 850 nm approximately. In the present embodiment, the wavelength of light emitted by the PPG sensor unit 122 may be selected as appropriate. In addition, the PPG sensor unit 122 includes, for example, a photodiode (photo detector or PD) and acquires the pulse wave signal by converting detected light intensity into an electric signal. The PPG sensor unit 122 may include a charge coupled device (CCD) sensor, a complementary metal oxide semiconductor (CMOS) sensor, or the like in place of the PD. The PPG sensor unit 122 may include an optical system mechanism such as a lens or a filter for detecting light from the measurement site 200 of the user.

The PPG sensor unit 122 can detect the pulse wave signal as temporally sequential data having a plurality of peaks as illustrated in FIG. 4. In the following description, the peak interval between a plurality of peaks appearing in the pulse wave signal as illustrated in FIG. 4 is referred to as a pulse rate interval (PPI). The value of the PPI can be acquired by processing the pulse wave signal detected by the PPG sensor unit 122. The value of each PPI is known to vary with time but substantially has normal distribution while the state of the target user is maintained constant. Thus, various heart rate variability (HRV) indexes for the physical state of the target user can be calculated by statistically processing a group of data of the PPI value (for example, by calculating the standard deviation of the PPI value). Details of the various HRV indexes will be described later.

In the present embodiment, the acquisition of the pulse wave signal is not limited to the above-described PPG method, but the pulse wave signal may be acquired by another method. For example, in the present embodiment, the sensor unit 120 may detect pulse wave by a laser Doppler blood current measurement method. The laser Doppler blood current measurement method measures blood current by exploiting a phenomenon as described below. Specifically, when the measurement site 200 of the target user is irradiated with a laser beam, a scattering ray subjected to Doppler shift is generated because a scattering material (red blood cells, mainly) in the blood vessel 202 of the target user is moving. Then, the scattering ray subjected to Doppler shift interferes with a scattering ray due to a tissue at rest in the measurement site 200 of the target user, and accordingly, intensity change in beat is observed. Thus, the laser Doppler blood current measurement method can measure blood current by analyzing the intensity and frequency of a beat signal.

In the following description, it is assumed that the sensor unit 120 of the wearable device 10 is provided with the PPG sensor unit 122 employing the above-described PPG method. In the present embodiment, an electrocardiogram (ECG) sensor unit (not illustrated) configured to detect the electrocardiogram of the target user through electrodes (not illustrated) bonded to the body of the target user may be provided in place of the PPG sensor unit 122. In this case, an R-R interval (RRI) as the beat interval of the heart can be acquired from the detected electrocardiogram, and a HRV index as a body index indicating the state of the body of the target user can be calculated from temporally sequential data of the RRI value.

In the present embodiment, the sensor unit 120 may be provided with a sweating sensor unit (not illustrated) in place of or together with the PPG sensor unit 122.

Specifically, sweating that occurs in a human typically mainly includes two kinds of sweating, namely, thermal sweating and mental sweating. The thermal sweating is performed to adjust body temperature. The mental sweating is caused by emotions of the human being, such as stress, delight, anger, sorrow, and pleasure, and instantaneously occurs in a small amount to a palm, a sole, or the like at room temperature as compared to the thermal sweating. For example, the mental sweating is sweating or the like caused by stress when performing a presentation. Such mental sweating is known to occur in a large amount when the sympathetic nerve is dominant, and is typically thought to be an index indicating emotion.

Thus, the above-described sweating sensor unit is mounted on the skin of the target user to detect voltage or resistance between two points on the skin, which is changed by sweating. In the present embodiment, the emotion of the target user can be estimated by acquiring information such as the amount of sweating, the frequency of sweating, and change in the amount of sweating based on sensing data acquired by the sweating sensor unit.

In addition, in the present embodiment, the sensor unit 120 may include, in place of or together with the PPG sensor unit 122, an image capturing apparatus (not illustrated) configured to capture facial expression of the target user. In this case, the image capturing apparatus detects, for example, ocular motion, the size of the pupil hole diameter, and the staring time of the target user. Muscles are thought to be in charge of the pupil hole diameter of a human being are affected by the sympathetic nerve and the parasympathetic nerve. Thus, in the present embodiment, the emotion of the target user, such as the states of the sympathetic nerve and the parasympathetic nerve of the target user can be estimated by sensing the pupil hole diameter of the target user by using the above-described image capturing apparatus.

The above-described image capturing apparatus may detect the posture of the target user. Posture is thought to affect, for example, the depth of breathing, and the depth of breathing is thought to be highly related with the stress state (stress degree) of the person. Thus, in the present embodiment, the posture of the target user can be detected by the image capturing apparatus, and the stress degree of the target user or the like can be estimated from the detected posture. Such an image capturing apparatus as described above may be installed around the target user as a device provided separately from the wearable device 10.

In the present embodiment, the sensor unit 120 may include other various biosensors (not illustrated) in place of or together with the PPG sensor unit 122. For example, the various biosensors may include one or a plurality of sensors directly or indirectly mounted on part of the body of the target user and configured to measure the brain wave, breathing, myopotential, skin temperature, and the like of the target user. More specifically, in the present embodiment, the emotional state (for example, the mood of the target user) of the target user can be estimated by analyzing sensing data obtained by a brain wave sensor unit (not illustrated) configured to measure the brain wave of the target user and detecting the kind (such as α wave or β wave) of the brain wave.

### - Motion sensor unit 124 -

The sensor unit 120 may include the motion sensor unit 124 for detecting the motion, in other words, motion state of the target user. The motion sensor unit 124 detects the motion state of the target user by acquiring sensing data indicating acceleration change that occurs along with motion of the target user. The motion state of the target user, which is acquired by the motion sensor unit 124 can be used to estimate the emotion of the target user. Specifically, the motion sensor unit 124 includes, for example, an acceleration sensor, a gyro sensor, and a geomagnetic sensor (all not illustrated).

The motion sensor unit 124 may be an image capturing apparatus (not illustrated) configured to capture an image of the target user. In this case, for example, motion of the target user can be captured based on the image captured by the image capturing apparatus, and thus the motion state of the target user can be detected by the image capturing apparatus. In addition, the motion sensor unit 124 may include an infrared sensor and an ultrasonic wave sensor (both not illustrated) that are capable of detecting motion of the target user. The image capturing apparatus, the infrared sensor, and the like may be installed around the target user as devices provided separately from the wearable device 10.

The sensor unit 120 may include a positioning sensor (not illustrated) in place of or together with the motion sensor unit 124. The positioning sensor is a sensor configured to detect the position of the target user on which the wearable device 10 is mounted, and specifically, may be a global navigation satellite system (GNSS) receiver or the like. In this case, the positioning sensor can generate sensing data indicating the latitude and longitude of the current position of the target user based on signals from GNSS satellites. In addition, in the present embodiment, a relative positional relation of the user can be detected from, for example, information of radio frequency identification (RFID), a Wi-Fi access point, and a wireless base station, and thus such a communication device can be used as the positioning sensor.

The sensor unit 120 may include, in place of or together with the motion sensor unit 124, a sound sensor (not illustrated) configured to detect speech voice of the target user or sound generated around the target user. For example, in the present embodiment, an extraction result obtained by extracting particular voice (for example, particular words spoken by the target user) from sound detected by the sound sensor may be used to estimate the emotion of the target user.

In addition, the sensor unit 120 may include a pressure sensor provided to a device (for example, a keyboard or a chair) used by the target user. For example, in the present embodiment, sensing data (for example, the frequency of typing on the keyboard or a seating time) acquired by the pressure sensor may be used to estimate the emotion of the target user. Such a pressure sensor as described above is installed as a device provided separately from the wearable device 10.

As described above, in the present embodiment, the sensor unit 120 may include various sensors. In addition, the sensor unit 120 may include a clock mechanism (not illustrated) configured to acquire an accurate time, and may associate acquired sensing data with a time at which the sensing data is acquired. The various sensors do not need to be provided in the sensor unit 120 of the wearable device 10 as described above, and for example, may be provided separately from the wearable device 10 or may be provided to a device used by the target user or the like.

### (Control unit 130)

The control unit 130 is provided in the wearable device 10 and can control each functional component of the wearable device 10 and acquire temporally sequential data of the PPI from the pulse wave signal output from the PPG sensor unit 122 described above. The control unit 130 is achieved by, for example, hardware such as a central processing unit (CPU), a read only memory (ROM), and a random access memory (RAM). Part of the function of the control unit 130 may be provided by the server 30 to be described later.

### (Communication unit 150)

The communication unit 150 is provided in the wearable device 10 and can transmit and receive information to and from external devices such as the server 30 and the terminal 50. In other words, the communication unit 150 can be regarded as a communication interface having a function to perform data transmission and reception. The communication unit 150 is achieved by communication devices such as a communication antenna, transmission and reception circuits, and ports.

### (Storage unit 160)

The storage unit 160 is provided in the wearable device 10 and stores, for example, computer programs and information for the control unit 130 described above to execute various kinds of processing, and information acquired through processing. The storage unit 160 is achieved by, for example, a nonvolatile memory such as a flash memory.

As described above, wearable devices of various schemes such as an armlet type and a HMD type can be employed as the wearable device 10. FIG. 5 illustrates exemplary appearance of the wearable device 10 according to the present embodiment. As illustrated in FIG. 5, the wearable device 10 is an armlet-type wearable device mounted on a wrist of the target user.

Specifically, as illustrated in FIG. 5, the wearable device 10 includes a band unit 12 in a belt shape, and a control unit 14. The band unit 12 is mounted, for example, around the wrist of the target user, and thus is formed of a material such as soft silicon gel to have a ring shape in accordance with the shape of the wrist. The control unit 14 is a part provided with the sensor unit 120, the control unit 130, and the like described above. The sensor unit 120 is provided at such a position that the sensor unit 120 contacts or faces the body of the target user when the wearable device 10 is mounted on part of the body of the target user.

### <3.2 Detailed configuration of server 30>

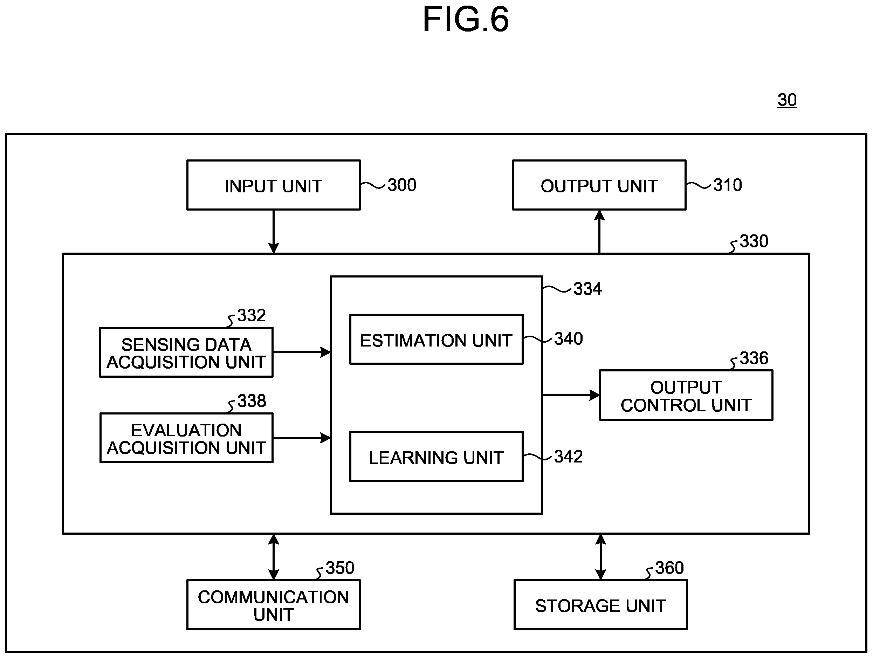

The following describes the configuration of the server 30 according to the present embodiment with reference to FIG. 6. FIG. 6 is a block diagram illustrating an exemplary configuration of the server 30 according to the present embodiment. As described above, the server 30 includes, for example, a computer. As illustrated in FIG. 6, the server 30 mainly includes an input unit 300, an output unit 310, a control unit 330, a communication unit 350, and a storage unit 360. Details of each functional component of the server 30 will be described below.

### (Input unit 300)

The input unit 300 receives input of data and commands from the user to the server 30. More specifically, the input unit 300 is achieved by, for example, a touch panel or a keyboard.

### (Output unit 310)

The output unit 310 includes, for example, a display, a speaker, a video output terminal, or a voice output terminal, and outputs various kinds of information to the user by image or voice.

### (Control unit 330)

The control unit 330 is provided in the server 30 and can control each block of the server 30. Specifically, the control unit 330 controls various kinds of processing performed in the server 30, such as estimation of the emotion of the target user and control in accordance with a result of the estimation. The control unit 330 is achieved by, for example, hardware such as a CPU, a ROM, and a RAM. The control unit 330 may execute part of the function of the control unit 130 of the wearable device 10. Details of the control unit 330 will be described later.

### (Communication unit 350)

The communication unit 350 is provided in the server 30 and can transmit and receive information to and from external devices such as the wearable device 10 and the terminal 50. The communication unit 350 is achieved by communication devices such as a communication antenna, transmission and reception circuits, and ports.

### (Storage unit 360)

The storage unit 360 is provided in the server 30 and stores, for example, computer programs for the control unit 330 described above to execute various kinds of processing, and information acquired through processing. More specifically, the storage unit 360 may store, for example, sensing data acquired from the wearable devices 10 mounted on a plurality of target users. The storage unit 360 is achieved by, for example, a magnetic recording medium such as a hard disk (HD), and a nonvolatile memory.

### <3.3 Detailed configuration of control unit 330>

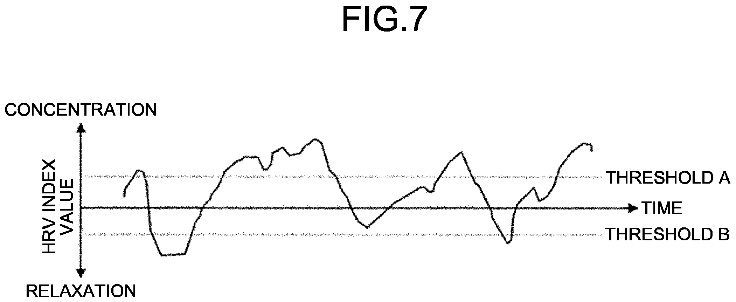

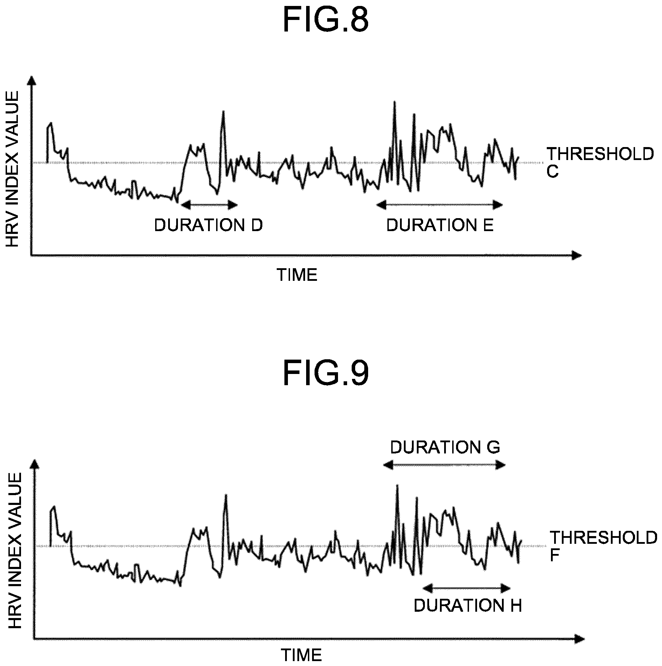

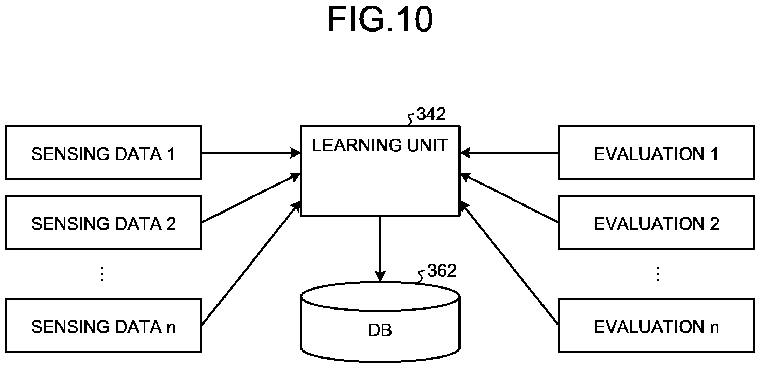

The following describes a detailed configuration of the control unit 330 according to the present embodiment with reference to FIGS. 6 to 10. FIGS. 7 and 8 are explanatory diagrams illustrating an exemplary HRV index obtained by an estimation unit 340 according to the present embodiment, and FIGS. 9 and 10 are explanatory diagrams of for describing exemplary machine learning performed by a learning unit 342 according to the present embodiment.

As illustrated in FIG. 6, the control unit 330 mainly includes a sensing data acquisition unit 332, a processing unit 334, an output control unit 336, and an evaluation acquisition unit 338. Details of each functional component of the control unit 330 will be described below.

### (Sensing data acquisition unit 332)

The sensing data acquisition unit 332 acquires sensing data output from the wearable device 10 or the like, and outputs the acquired sensing data to the processing unit 334 to be described later.

### (Processing unit 334)

The processing unit 334 processes sensing data output from the sensing data acquisition unit 332, and estimates the state (emotion) of the target user. In addition, the processing unit 334 can estimate the state (emotion) of the target user or the like after the sensing data is acquired. Specifically, the processing unit 334 functions as the estimation unit 340 and the learning unit 342 to achieve these above-described functions.

### - Estimation unit 340 -

The estimation unit 340 estimates, for example, the state (emotion) of the target user based on sensing data acquired from the wearable device 10. Specifically, the estimation unit 340 estimates the current excitation degree, stress degree, concentration degree, relaxation degree, awareness, sleepiness, and the like of the target user based on, for example, the sensing data, in other words, biological signals of the pulse rate, sweating, and the like.

More specifically, the estimation unit 340 acquires temporally sequential data of the PPI value, which is the interval between peaks, from a pulse wave signal as illustrated in FIG. 4. In addition, the estimation unit 340 calculates various HRV indexes by performing statistical processing by using the PPI temporally sequential data. Examples of the HRV indexes include a root mean square successive difference (RMSSD), a standard deviation of the normal to normal interval (SDNN), and an LF/HF. These HRV indexes can be handled as indexes indicating the emotion of the target user as described above.

Specifically, the RMSSD is the square root of the average value of the square of the difference between PPI values adjacent to each other on temporal sequence of the PPI. The RMSSD is thought to serve as an index indicating the stress state of the vagal nerve, which is one of the cranial nerves.

The SDNN is the standard deviation of a group of data of the PPI value in a predetermined duration. The SDNN is thought to serve as an index indicating the activity status of the autonomic nerve system including the sympathetic nerve and the parasympathetic nerve.

The LF/HF is the ratio of the power spectrum of a low frequency component (for example, 0.04 to 0.15 Hz) of the PPI temporally sequential data relative to the power spectrum of a high frequency component (for example, 0.15 to 0.4 Hz) thereof. The high frequency component (HF) is thought to correspond to breathing variation and the low frequency component (LF) is thought to correspond to Mayer wave as pressure blood variation. In addition, it is thought that the high frequency component corresponding to breathing variation reflects the parasympathetic nerve and the low frequency component reflects the sympathetic nerve. Thus, the LF/HF is thought to serve as an index indicating the balance between the sympathetic nerve and the parasympathetic nerve, and the LF/HF can be thought to indicate the balance between the sympathetic nerve and the parasympathetic nerve, in other words, the state of the autonomic nerve, more specifically, the concentration degree (stress) and relaxation degree of the target user.

The following describes a case in which temporally sequential data of the LF/HF is acquired as a HRV index by the above-described estimation unit 340. In this case, the estimation unit 340 can obtain, for example, temporally sequential data of the HRV index as illustrated in FIG. 7. Threshold A illustrated in FIG. 7 is a threshold with which the concentration degree of the target user can be estimated to be high, in other words, the concentration degree of the target user can be estimated to be high when the HRV index is higher than Threshold A. Threshold B illustrated in FIG. 7 is a threshold with which the target user can be estimated to be relaxed, in other words, the target user can be estimated to be relaxed when the HRV index is lower than Threshold B. Thus, in the present embodiment, the estimation unit 340 can estimate the concentration degree of the target user and the like by, for example, comparing the obtained HRV index with Thresholds A and B.

The estimation unit 340 may estimate, for example, the concentration degree of the target user by comparing the high and low frequency components to determine whether the activation degree of the sympathetic nerve or the activation degree of the parasympathetic nerve is dominant.

The estimation unit 340 may perform the estimation by using sensing data indicating motion of the target user, which is obtained by the motion sensor unit 124 included in the sensor unit 120. For example, assume that the estimation unit 340 acquires temporally sequential data of the HRV index as illustrated in FIG. 8. Threshold C illustrated in FIG. 8 is assumed to be a threshold with which the concentration degree of the target user can be estimated to be high. Thus, the estimation unit 340 can estimate the concentration degree of the target user to be high in Duration D and Duration E in each of which the HRV index is higher than Threshold C illustrated in FIG. 8. However, the estimation unit 340 does not estimate the concentration degree of the target user to be high when having detected from the sensing data obtained by the motion sensor unit 124 that the target user is performing active motion in Duration D. Specifically, the sympathetic nerve is normally active when active motion is performed. Thus, in the present embodiment, when having detected active motion based on the sensing data obtained by the motion sensor unit 124, the estimation unit 340 presumes that the HRV index has become high due to the motion, and does not estimate the concentration degree of the target user to be high. When having detected that the target user is at rest from the sensing data obtained by the motion sensor unit 124 in Duration E, the estimation unit 340 presumes that the HRV index has become high due to concentration, in other words, estimates the concentration degree of the target user to be high.

In the present embodiment, the estimation unit 340 is not limited to estimation based on the HRV index obtained from the pulse wave signal as described above, but may estimate the emotion of the target user based on the number of pulsebeats that can be acquired from the pulse wave signal. For example, when having detected from the sensing data that the target user is at rest but the number of pulsebeats has become larger than that at normal time (the interval of pulsebeats has become shorter than that at normal time), the estimation unit 340 estimates that the sympathetic nerve is dominant and stress is high in the target user. When having detected from the sensing data that the number of pulsebeats of the target user has become smaller than that at normal time (the interval of pulsebeats has become longer than that at normal time), the estimation unit 340 estimates that the parasympathetic nerve is dominant and sleepiness is high in the target user.

In addition, in the present embodiment, the estimation unit 340 may estimate the emotion of the target user by using sensing data indicating the posture and motion of the target user, which is detected by any of the other various sensors included in the sensor unit 120, for example, the image capturing apparatus (not illustrated). More specifically, when the image capturing apparatus has detected that the target user is seated in a posture inclined forward, the estimation unit 340 may estimate that the target user is in concentration. When the image capturing apparatus has detected that the target user is performing leg shaking (periodical shaking of a body part such as leg while seated), the estimation unit 340 may estimate that the target user is not in concentration or is in bad mood. In addition, when the image capturing apparatus has detected that the target user has performed no body motion for a long time, the estimation unit 340 may estimate that the target user suffers fatigue or is depressed.

In addition, the estimation unit 340 may estimate the emotion of the target user by detecting the posture and motion of the target user as described above by using sensing data acquired by a pressure sensor (not illustrated) provided to a chair or the like used by the target user.

The estimation unit 340 may estimate the emotions of one or a plurality of target users by using sensing data obtained by the sound sensor (not illustrated) included in the sensor unit 120. More specifically, when the sound sensor has detected that the frequency of conversation is high, the estimation unit 340 estimates that members of a group including the users are in concentration. When the sound sensor has detected that the frequency of conversation is low or has detected yawn, the estimation unit 340 estimates that some members of a group including the users are bored or sleepy and the concentration degree is low. In the present embodiment, the emotion of the target user may be estimated based on the sound volume of speech by the target user, which is obtained from the sensing data obtained by the sound sensor, and spoken contents.

The estimation unit 340 may estimate the state (emotion) of the target user based on a state tendency (emotional tendency) of the target user, which is obtained through learning by the learning unit 342 to be described later. The tendency of the state (emotion) of a human being differs between individuals, and thus the learning unit 342 can acquire information of the state tendency of the target user by using sensing data of the target user in advance, and the estimation can be performed based on the acquired state tendency, which improves the accuracy of the estimation. In addition, the estimation unit 340 may estimate a future state of the target user based on the state tendency obtained by the learning unit 342.

### - Learning unit 342 -

As described above, the learning unit 342 learns the state tendency (emotional tendency) of the target user by using sensing data acquired in the past. Then, the state tendency of the target user, which is obtained through the learning by the learning unit 342 can be used for the estimation of the state (emotion) of the target user by the estimation unit 340.

For example, in the estimation of the concentration degree of the target user based on the HRV index as described above, the concentration degree of the target user can be objectively estimated through comparison with a threshold, but may be different from subjective observation by the target user. More specifically, assume that the estimation unit 340 has acquired temporally sequential data of the HRV index as illustrated in FIG. 9. Threshold F illustrated in FIG. 9 is assumed to be a threshold with which the concentration degree of the target user can be estimated to be high. Thus, since the HRV index is higher than Threshold F, the target user can be objectively estimated to be in concentration in Duration G in FIG. 9. However, in reality, the target user may feel to be in concentration in Duration H in FIG. 9, which is shifted from Duration G. Thus, in the present embodiment, the concentration state of the target user can be more accurately estimated by adjusting the difference between objective estimations based on the HRV index and subjective observation by the target user.

Specifically, while the wearable device 10 is mounted on the target user, the target user plays a game such as a puzzle game, which is likely to cause concentration. During the game, the sensing data acquisition unit 332 acquires the pulse wave signal of the target user as sensing data. In this case, the concentration degree is evaluated by the target user and acquired by the evaluation acquisition unit 338 to be described later. Then, the learning unit 342 determines, based on the HRV index obtained by processing the sensing data, whether a duration in which the target user is estimated to be in concentration and a duration in which the target user evaluates that the target user is in concentration match each other. In addition, when the two durations do not match each other, the learning unit 342 changes the value of Threshold F with which the concentration degree of the target user is estimated to be high so that the durations match each other. In this manner, in the present embodiment, the difference between objective estimations based on the HRV index and subjective observation by the target user is adjusted to more accurately estimate the concentration state of the target user. The present embodiment is not limited to change of the value of Threshold F, but another method such as provision of a temporal offset may be selected. In addition, the present embodiment is not limited to acquisition of evaluation of the concentration degree by the target user, but for example, an instruction of the timing of device control desired by the target user (for example, an instruction to perform device control earlier or later than a concentration duration estimated based on the HRV index) may be acquired. In this case, the output control unit 336 to be described later performs output control based on the instruction, and thus control more preferable for the target user is performed.

The learning unit 342 may be a supervised learning machine by, for example, support vector regression or deep neural network. As illustrated in FIG. 10, sensing data acquired from the wearable device 10 in the past and evaluation (for example, the concentration degree) by the target user when the sensing data is acquired are input to the learning unit 342 as a teacher signal and an input signal, respectively. Then, the learning unit 342 performs machine learning on the relation between these signals in accordance with a predetermined rule. As a result, the learning unit 342 can establish a database (DB) 362 storing relation information indicating the relation (such as emotional tendency) between the sensing data and the evaluation by the target user. In addition, the estimation unit 340 estimates the emotion of the target user or the like by using the DB 362 obtained by machine learning as described above. In the present embodiment, the learning unit 342 may be a semi-supervised learning machine or a weakly supervised learning machine.

The learning unit 342 may establish the DB 362 by learning the tendency of the continuation time of the emotion of the target user based on sensing data or the like. Then, when sensing data is acquired next time, the estimation unit 340 may expect, by using the DB 362, future emotion of the target user after the sensing data is acquired. For example, when the target user is estimated to be in concentration once, the learning unit 342 learns, based on a plurality of pieces of sensing data, the tendency of the continuation time in which the concentration can be continuously maintained. Then, when concentration of the target user is estimated as the current state, the estimation unit 340 predicts a time for which the target user can continue the concentration thereafter based on the continuation time tendency learned by the learning unit 342.

### (Output control unit 336)

The output control unit 336 performs output corresponding to an estimation result obtained by the estimation unit 340. More specifically, as output corresponding to the estimation result, the output control unit 336 controls the terminal 50 to be described later and outputs information corresponding to the estimation result to the terminal 50.

### (Evaluation acquisition unit 338)

As described above, the evaluation acquisition unit 338 acquires, for example, evaluation of the state (emotion) of the target user by the target user and outputs the acquired evaluation to the processing unit 334. The evaluation acquisition unit 338 may acquire target user's evaluation of the estimation result obtained by the estimation unit 340.

The server 30 may include a context acquisition unit (not illustrated) configured to acquire attribute information such as the schedule, sex, and age of the target user, and the like. Such information can be used in the estimation at the estimation unit 340 and the learning at the learning unit 342, thereby further improving the accuracy of estimation of the state (emotion) of the user.

### <3.4 Detailed configuration of terminal 50>

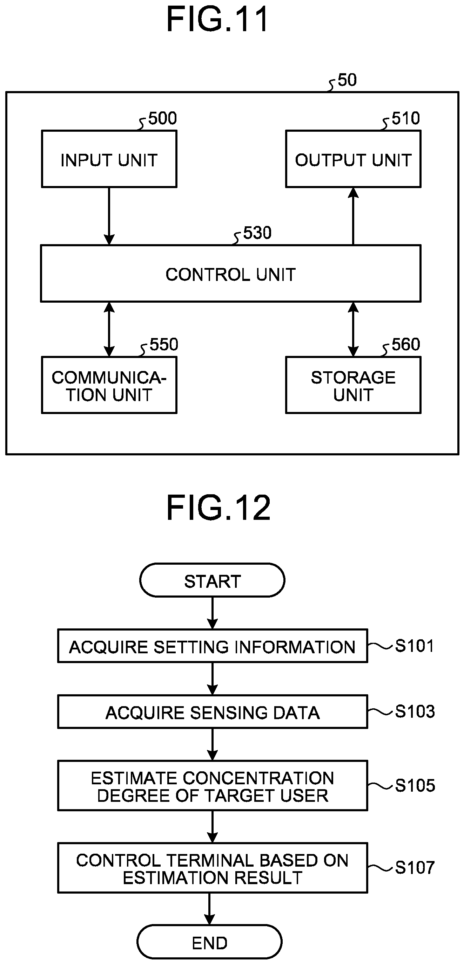

The following describes the configuration of the terminal 50 according to the present embodiment with reference to FIG. 11. FIG. 11 is a block diagram illustrating an exemplary configuration of the terminal 50 according to the present embodiment. As described above, the terminal 50 is a device such as a tablet, a smartphone, a cellular phone, a laptop PC, a notebook PC, or an HMD. As illustrated in FIG. 11, the terminal 50 mainly includes an input unit 500, an output unit 510, a control unit 530, a communication unit 550, and a storage unit 560. Each above-described functional component of the terminal 50 is common to a functional component having an identical name in the server 30, and thus description of the functional component will be omitted in the following.

### <3.5. Information processing method>

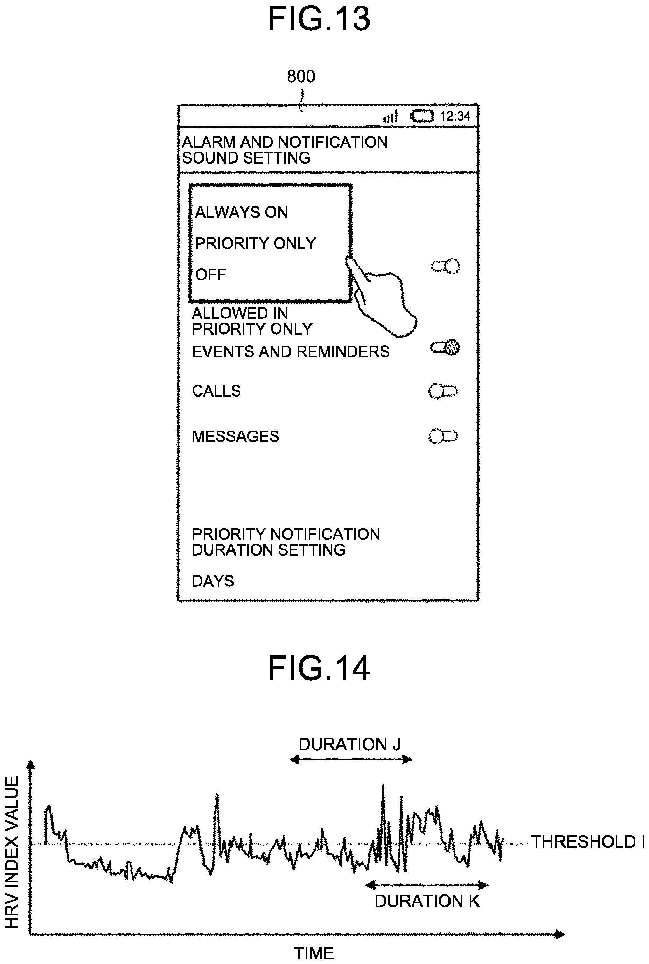

The above description is made on detailed configurations of the wearable device 10, the server 30, and the terminal 50 according to the present embodiment. The following describes an information processing method according to the present embodiment with reference to FIGS. 12 and 13. FIG. 12 is a flowchart for describing an exemplary information processing method according to the present embodiment, and FIG. 13 is an explanatory diagram illustrating an exemplary setting screen 800 of the terminal 50 according to the present embodiment.

As illustrated in FIG. 12, the information processing method according to the present embodiment includes a plurality of steps S101 to S107. The following describes details of each step included in the information processing method according to the present embodiment.

### (Step S101)

The server 30 acquires setting information related to setting of the terminal 50 as a smartphone used by the target user. Specifically, the target user sets in advance an operation desired for the smartphone used by the target user when the concentration degree of the target user has increased. For example, the target user sets, on the setting screen 800 displayed on a display unit (not illustrated) of the smartphone as illustrated in FIG. 13, an operation of the alarm of the smartphone when the concentration degree of the target user has increased. As illustrated in FIG. 13, the setting screen 800 displays three options, namely, an option for the alarm to sound whenever a notification is received, an option for the alarm to sound only when an important notification is received, and an option for the alarm not to sound whenever a notification is received. The target user performs a selection operation on any of the options to set an operation of the alarm of the smartphone when the concentration degree of the target user has increased. In the following description, it is assumed that the target user has selected the option for the alarm to sound only when an important notification is received.

### (Step S103)

Subsequently, the above-described wearable device 10 according to the present embodiment is mounted on the target user, and then acquires the pulse wave signal of the target user as sensing data and outputs the sensing data to the server 30. The server 30 acquires the sensing data from the wearable device 10.

### (Step S105)

The server 30 estimates the emotion of the target user, in this example, the concentration degree thereof based on the acquired sensing data. The method of estimating the emotion of the target user is described above, and thus detailed description thereof will be omitted in the following.

### (Step S107)

Then, when the concentration degree of the target user is estimated to be high, the server 30 controls the smartphone to set the alarm of the smartphone not to sound when a notification other than an important notification is received.

Thereafter, when having estimated that the concentration degree of the target user has decreased based on the sensing data acquired from the wearable device 10, the server 30 controls the smartphone to change setting so that the alarm sounds whenever a notification is received. In the present embodiment, the setting of the smartphone is not limited to setting as described above. For example, in the present embodiment, the smartphone may be set to a manner mode in which the smartphone vibrates without outputting sound when a notification is received, or may be set to an airplane mode in which the communication function of the smartphone is turned off.

As described above, in the present embodiment, when the concentration state of the target user is estimated to be high, the smartphone (terminal 50) used by the target user is controlled not to sound the alarm upon an incoming alert. As a result, the target user can continue work in concentration, which can improve the performance of the target user. In other words, in the present embodiment, not only the emotion of the target user is estimated but also needs of the target user are specified in accordance with the estimated emotion and the terminal 50 is controlled in accordance with the specified needs. Thus, according to the present embodiment, the target user can create a situation preferable for the target user without taking any action in particular.

### <3.6 Modification 1>

As described above, the server 30 acquires attribute information such as the schedule, sex, and age of the target user and uses the information in the estimation at the estimation unit 340 and the learning at the learning unit 342, thereby further improving the accuracy of estimation of the emotion of the target user. Thus, the following describes, as Modification 1 of the present embodiment, an example in which the schedule of the target user is acquired to further improve the accuracy of estimation of the emotion of the target user with reference to FIG. 14. FIG. 14 is an explanatory diagram for describing Modification 1 of the embodiment.

More specifically, assume that the server 30 has acquired temporally sequential data of the HRV index of the target user as illustrated in FIG. 14. Threshold I illustrated in FIG. 14 is assumed to be a threshold with which the concentration degree of the target user is estimated to be high. In addition, it is assumed that the server 30 acquires in advance information (schedule) indicating that the target user is scheduled to produce a document in Duration J in FIG. 14. In such a case, the server 30 estimates the target user to be in concentration in Duration K in FIG. 14 based on the information that the document production, in which the HRV index is higher than Threshold I and the concentration degree of the target user is expected to increase, is scheduled in Duration J. In this manner, in the present modification, the estimation of the emotion of the user can be further improved by referring to not only the HRV index but also the schedule of the target user.

In the present modification, not only the schedule of the target user is used in the estimation of the emotion of the target user, but also a threshold used to estimate the emotion may be controlled in accordance with the schedule of the target user. Specifically, the target user is scheduled to produce documents in Duration J in FIG. 14. Thus, the server 30 may presume that the concentration degree of the target user is likely to increase in Duration J, and may decrease the value of Threshold I with which the concentration degree is estimated to be high in Duration J so that the target user is more likely to be estimated to be in concentration. Accordingly, the smartphone used by the target user is likely to be controlled not to sound the alarm upon an incoming alert when the target user is at least slightly in concentration, and the target user can continue work in deeper concentration.

As described above, in the present modification, the accuracy of estimation of the emotion of the target user and control can be further improved by using information such as the schedule of the target user. As a result, in the present modification as well, the target user can create a situation preferable for the target user without taking any action in particular.

### <3.7 Modification 2>

In the above-described first embodiment, the terminal 50 (smartphone) used by the target user is controlled in accordance with the estimated emotion of the target user, but in the present embodiment, another terminal 50 may be controlled. For example, the other terminal 50 is the display lamp 700 (refer to FIG. 16) installed near the target user, or a PC used by another user included in a group to which the target user belongs. Exemplary control of such a terminal 50 will be described as Modification 2 assumed for use in an office or the like.

For example, the target user having an increased concentration degree while performing work or the like often feels unpleasant, for example, when being spoken to by any other user, which could be a factor of interruption of the work. Thus, in the present modification, when the concentration degree of the target user has increased, the display lamp 700 is controlled to notify, to any other user, a recommended action or the like recommended on how to deal with the target user. Specifically, in the present modification, not only the emotion of the target user is estimated, but also needs of the target user are specified in accordance with the estimated emotion and output is performed toward any other user in accordance with the specified needs. Thus, according to the present modification, the target user can have an environment preferable for the target user without taking any action in particular.

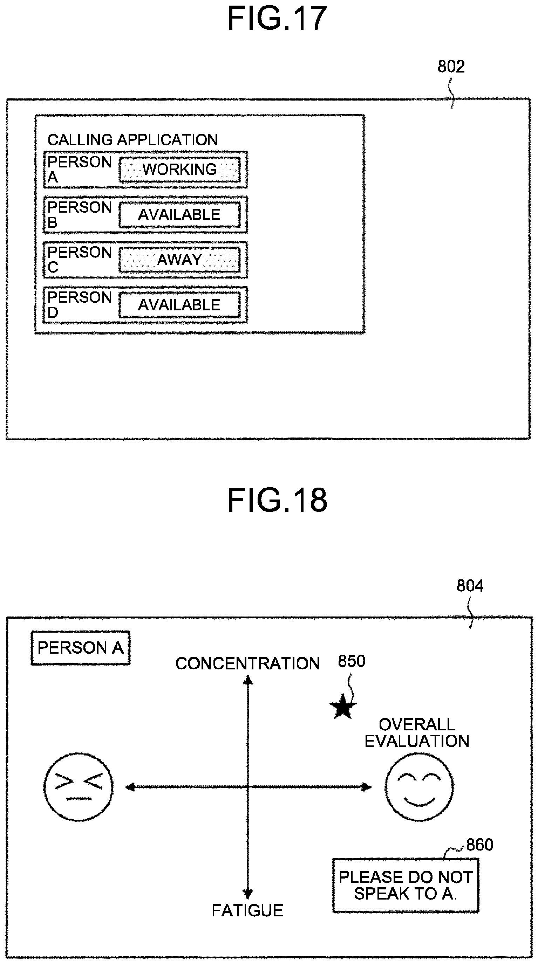

The following describes details of the present modification with reference to FIGS. 15 to 17. FIG. 15 is a flowchart for describing an exemplary information processing method according to Modification 2 of the present embodiment. FIG. 16 is an explanatory diagram illustrating an exemplary display lamp 700 according to Modification 2 of the present embodiment, and FIG. 17 is an explanatory diagram illustrating an exemplary display screen 802 according to Modification 2 of the present embodiment.

As illustrated in FIG. 15, the information processing method according to the present modification includes a plurality of steps S201 to S205. Steps S201 and S203 in FIG. 15 in the present modification are same as steps S103 and S105 in FIG. 12, and thus only step S205 will be described in the following without description of steps S201 and S203.

### (Step S205)

When the concentration degree of the target user is estimated to be high, the server 30 controls to turn on the display lamp 700 installed near the target user as illustrated in FIG. 16. For example, a phrase such as "Working. Do not disturb" is written on the display lamp 700, and when turned on, the display lamp 700 notifies, to another user coming closer to the target user, a recommended action recommended as on how to deal with the target user, such as not to speak to the target user. As a result, when the target user is in concentration, the display lamp 700 is turned on to prevent any other user from speaking to the target user, and thus the target user can continue working in deeper concentration.

As described above, according to the present modification, the display lamp 700 is controlled in accordance with the estimated emotion of the target user so that a situation preferable for the target user can be created.

The present modification is not limited to control of the display lamp 700 as described above, but for example, a PC (terminal 50) used by another user included in a group to which the target user belongs may be controlled. In such a case, for example, as illustrated in FIG. 17,the display screen 802 of a calling application displayed on a display unit (not illustrated) of a PC used by each user displays that the target user is "working" in accordance with estimation that the target user (in FIG. 17, "Person A") is in concentration. The present modification is not limited to display on the display screen 802 of the calling application, but the display may be performed on the display screen of a schedule management application, or another user may be notified through an electronic mail that the target user is "working".

### <3.8 Modification 3>

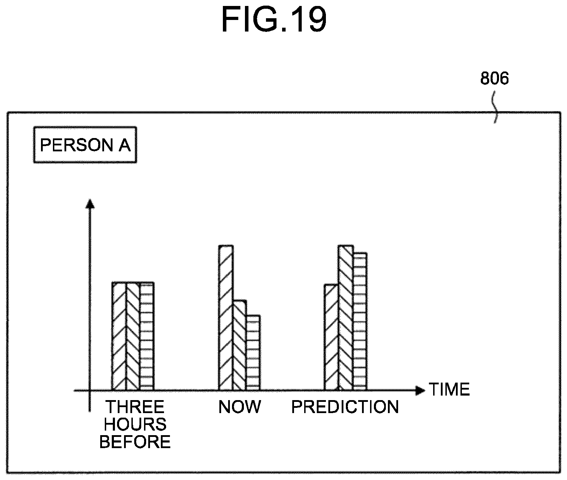

In the above-described first embodiment, the concentration degree is estimated as the emotion of the target user. However, in the present embodiment, not only the concentration degree but also the fatigue degree, sleepiness, or the like of the target user may be estimated. Thus, an example in which the fatigue degree or sleepiness of the target user is estimated and a notification in accordance with a result of the estimation is performed toward any other user will be described below with reference to FIGS. 18 and 19 as Modification 3 assumed for use in an office or the like. FIG. 18 is an explanatory diagram illustrating an exemplary display screen 804 according to Modification 3 of the present embodiment, and FIG. 19 is an explanatory diagram illustrating an exemplary display screen 806 according to Modification 3 of the present embodiment.

For example, in the present modification, the server 30 estimates the concentration degree or fatigue degree of the target user and notifies, to the terminal 50 such as a PC used by an administrator (user) who manages the target user in work, a result of the estimation and a recommended action recommended as on how to deal with the target user. More specifically, the server 30 causes a display unit (not illustrated) of the PC used by the administrator to display the display screen 804 as illustrated in FIG. 18. On the display screen 804, the estimated emotion of the target user (in FIG. 18, Person A) is illustrated with a marker 850 on XY coordinates. Specifically, the X axis of the XY coordinates represents the mood of the target user, indicating that, for example, the mood is better at a position further on the right side in FIG. 18 and the mood is worse at a position further on the left side in FIG. 18. The Y axis of the XY coordinates represents the concentration degree or fatigue degree of the target user, indicating that, for example, the concentration degree is higher at a position further on the upper side in FIG. 18 and the fatigue degree is higher at a position further on the lower side in FIG. 18. When the estimated emotion of the target user is displayed as the marker 850 on such XY coordinates, the administrator can easily understand the emotion of the target user. As described above, the mood of the target user can be estimated by, for example, analyzing sensing data obtained by a brain wave sensor included in the sensor unit 120 of the wearable device 10.

In addition, on the display screen 804, the server 30 not only displays the estimated emotion of the target user but also displays, as a comment 860, a recommended action recommended as on how to deal with the target user in accordance with the estimation result. For example, in FIG. 18, a phrase such as "Please do not speak to A" is displayed as the comment 860 upon estimation that the target user is in concentration. Thus, the administrator can understand, based on the comment 860, the recommended action recommended as on how to deal with the target user and perform handling such as not to speak to the target user.

For example, when the fatigue degree of the target user is estimated to be high, the server 30 may display, on the PC of the administrator, a phrase such as "Speak to A.", "Recommend a break.", "Give consultation.", or "Adjust a workload.".

The server 30 may not only estimate the current emotion of the target user but may predict the emotion of the target user thereafter and notify a result of the prediction to the administrator or the like. More specifically, the server 30 causes the display unit (not illustrated) of the PC used by the administrator to display the display screen 806 as illustrated in FIG. 19. The estimated concentration degree, fatigue degree, and sleepiness of the target user (in FIG. 19, Person A) are illustrated as three bar graphs on the display screen 806. Specifically, the concentration degree, the fatigue degree, and the sleepiness correspond to the bar graphs, respectively, from the left side in FIG. 19. In addition, the display screen 806 displays, in a temporally sequential manner from the left side in FIG. 19, the concentration degree, fatigue degree, and sleepiness of the target user three hours before, the current concentration degree, fatigue degree, and sleepiness of the target user, and the expected concentration degree, fatigue degree, and sleepiness of the target user in the future. In FIG. 19, a higher bar graph indicates that the concentration degree, the fatigue degree, or the sleepiness is higher. The administrator can easily understand the past, current, and future emotions of the target user by checking the display screen 806. In addition, the administrator can accept that a recommended action recommended as handling of the target user, which is notified from the server 30 is an appropriate action by referring to the past, current, and future emotions of the target user.

The present modification is not limited to notification of a recommended action to the PC used by the administrator, but the recommended action may be notified to a terminal 50 used by, for example, another user or a family member around the target user.

As described above, according to the present embodiment and modifications, a situation preferable for the target user can be created by estimating the emotion of the target user, visualizing a result of the estimation for display, and performing notification and control in accordance with the estimation result. For example, when the target user is not aware of the state (emotion) of himself/herself or has difficulties in notifying this state to others, any other user around the target user can recognize the state of the target user and perform an appropriate action for the target user according to the present embodiment. In particular, recently, it has been difficult to manage mental health of workers at an office due to significant increase of a workload caused by shortage of labor. In such a situation, before each worker becomes fully exhausted, an administrator of the office can know a presage thereof and take an appropriate action by using the information processing system 1 according to the present embodiment, thereby excellently managing the mental health of each worker.

### <<4. Second embodiment>>

In the above-described first embodiment, the emotion of each target user is estimated. However, the embodiment of the present disclosure is not limited to such estimation, but the emotions of a plurality of target users, in other words, the emotion of a group may be estimated. Thus, in a second embodiment of the present disclosure describes below, the emotion of a group including a plurality of target users is estimated, and output is performed in accordance with a result of the estimation.

In an important scene such as business, work is often cooperatively performed by a plurality of members. However, it is difficult to objectively evaluate the state of such a group of a plurality of members. Specifically, in brainstorming performed by a plurality of members, whether the brainstorming is effectively performed can be evaluated based on the number of statements or the like, but it has been difficult to verify, for example, a synergy effect due to the configuration of the members. In particular, the success of brainstorming often depends not only on the ability of each member but also on the configuration of the members. Specifically, for successful brainstorming, it is typically important to produce an atmosphere in which each member can give ideas without feeling uneasy for other members. Thus, the configuration of members is important to create such an atmosphere. Thus, in the present embodiment describes below, the emotion of a group in brainstorming, which is caused by the synergy effect of a group is estimated, and a notification related to the future progress of brainstorming is output in accordance with a result of the estimation. In the following description, brainstorming means a discussion performed in expectation of induction of new ideas by a chain effect among members when giving ideas within the group.

Specifically, in the present embodiment, the concentration degrees of at least some target users included in a group in brainstorming are estimated by using the above-described information processing system 1, and the concentration degree of the group is estimated based on a result of the estimation. In addition, in the present embodiment, whether to continue the brainstorming is notified to, for example, a facilitator of the brainstorming based on the estimation result. In conventional cases, the facilitator subjectively determines whether to continue brainstorming based on the content of the brainstorming or the like. However, in the present embodiment, the concentration degree of a group performing brainstorming is estimated, and whether to continue the brainstorming is objectively determined in accordance with a result of the estimation. As a result, the facilitator can appropriately proceed the brainstorming and derive the maximum effect of the brainstorming. The following describes details of the present embodiment.

In the present embodiment, the configurations of the information processing system 1, the wearable device 10, the server 30, and the terminal 50 are common to those in the first embodiment, and the description of these configurations in the first embodiment can be referred to. Thus, in the following, description of detailed configurations of the information processing system 1, the wearable device 10, the server 30, and the terminal 50 will be omitted. In the following description, it is assumed that a group performing brainstorming includes two target users and also includes other users. In the present embodiment, the group only needs to include at least two target users, and may include three or more target users or may include only two target users and no other users.

### <4.1 Information processing method>

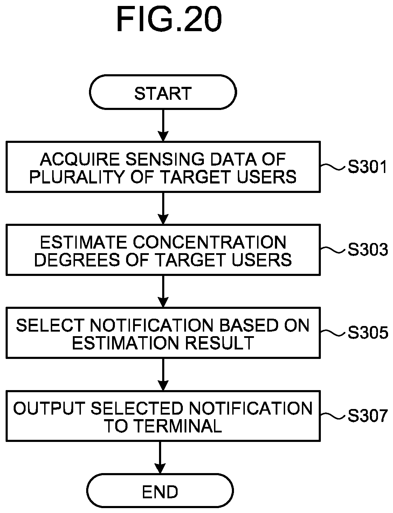

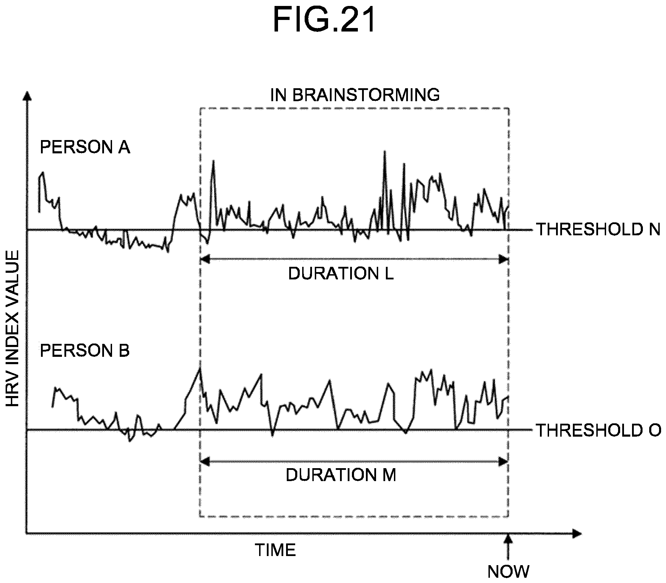

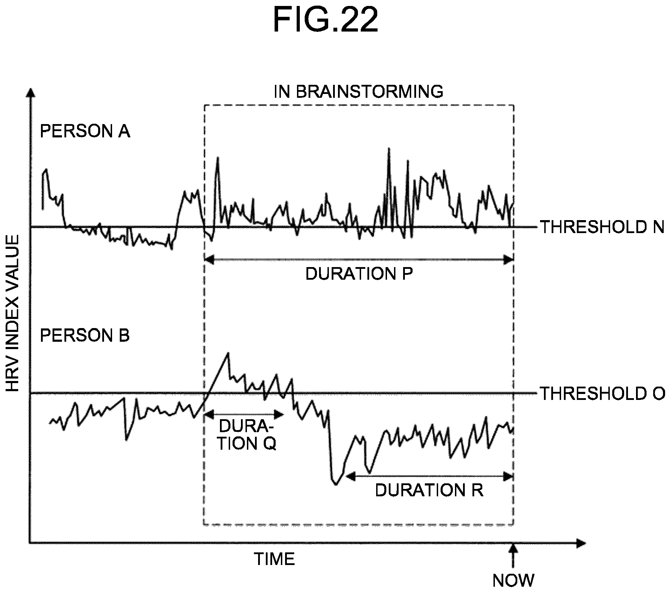

The following describes the information processing method according to the present embodiment with reference to FIGS. 20 to 22. FIG. 20 is a flowchart for describing an exemplary information processing method according to the present embodiment, and FIGS. 21 and 22 are explanatory diagrams for describing exemplary information processing according to the present embodiment. FIGS. 21 and 22 illustrate temporally sequential data of the HRV indexes of the target users (in FIGS. 21 and 22, Persons A and B) in brainstorming. Specifically, temporally sequential data of the HRV index of Person A is illustrated at the upper part in each of FIGS. 21 and 22, and temporally sequential data of the HRV index of Person B is illustrated at the lower part in each of FIGS. 21 and 22. In addition, the temporally sequential data of each HRV index is provided with Threshold N or Threshold O as a reference for estimation that the target user is in concentration. Accordingly, when the HRV index is higher than Threshold N or O, the target user is estimated to be in concentration.

As illustrated in FIG. 20, the information processing method according to the present embodiment includes a plurality of steps S301 to S307. The following describes details of each step included in the information processing method according to the present embodiment.

### (Step S301)

The above-described wearable device 10 is mounted on each target user participating in brainstorming, and each wearable device 10 acquires the pulse wave signal or the like of the target user as sensing data, and outputs the sensing data to the server 30. The server 30 acquires the sensing data from each wearable device 10.

### (Step S303)

The server 30 estimates the concentration degree of each target user based on the acquired sensing data. Since the concentration degree estimation method is described above, detailed description thereof will be omitted in the following.

### (Step S305)

The following first describes a case in which, in brainstorming, Person A can be estimated to be in concentration in Duration L, and similarly, Person B can be estimated to be in concentration in Duration M as illustrated in FIG. 21. In such a case, the server 30 can estimate that each target user is in concentration, and thus presumes that any other user participating in the brainstorming is in concentration, and determines that the brainstorming is effectively performed. In addition, the server 30 selects, based on the determination, to perform notification that suggests continuation of the brainstorming to the facilitator.

The following describes a case in which Person A and Person B can be estimated to be in concentration in Duration P and Duration Q in brainstorming but Person B can be estimated not to be in concentration or to be sleepy in Duration R as illustrated in FIG. 22. In such a case, since the concentration degree of Person B participating in the brainstorming is low, the server 30 presumes that the concentration degree of any other participating user is low. In addition, the server 30 determines based on the presumption that it is difficult to effectively proceed the brainstorming from now, and selects to perform notification that suggests cancellation or interruption of the brainstorming to the facilitator. When any target user estimated to be extremely excited is detected, the server 30 may select to perform notification that suggests cancellation or interruption of the brainstorming to the facilitator because the occurrence of a fight or the like may be anticipated due to excitation.

### (Step S307)

In accordance with the selection at step S305 described above, the server 30 controls, for example, the terminal 50 used by the facilitator to display, on a screen, notification that suggests continuation, cancellation, or interruption of the brainstorming.

As described above, in the present embodiment, the concentration degrees of a plurality of target users in brainstorming are estimated, and the concentration degree of this group is estimated based on a result of the estimation. In addition, in the present embodiment, whether to continue the brainstorming is notified to, for example, the facilitator of the brainstorming in accordance with the estimation result. Thus, according to the present embodiment, whether to continue the brainstorming can be objectively determined instead of subjective determination by the facilitator or the like. As a result, the facilitator can appropriately proceed the brainstorming and derive the maximum effect of the brainstorming.

The present embodiment is not limited to use in brainstorming but may be used at, for example, an event site. Specifically, the above-described wearable device 10 is mounted on each participant of an event to estimate emotion of the participant. When each participant is moderately excited, it is determined that the participant is enjoying the event, and the server 30 suggests continuation of the current progress content to a facilitator of the event. When each participant is not excited, it is determined that the participant is not enjoying the event, and the server 30 suggests change of the content of the event to the facilitator of the event. In this manner, according to the present embodiment, the content can be changed in accordance with the emotion of each participant of the event, and thus the participant can be provided with a more enjoyable event. In addition, when having detected a participant being extremely excited, the server 30 may suggest, for example, guiding the participant to a separate room to the facilitator. When the facilitator guides the participant to a separate room in accordance with this suggestion and recommends a break, it is possible to prevent event disorder that would occur due to extreme excitation.

### <4.2 Modification 1>

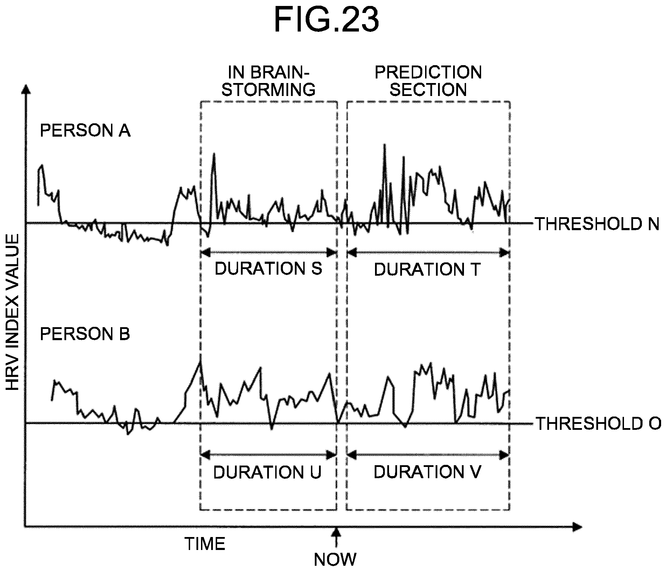

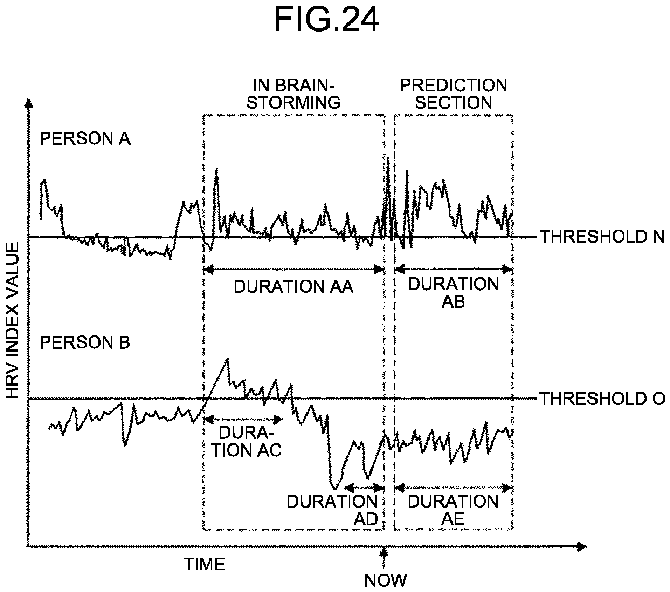

The server 30 can predict the concentration degree of the target user in the future based on sensing data, and thus may select whether to continue the above-described brainstorming based on the prediction in Modification 1 of the present embodiment describes below. The following describes the present modification with reference to FIGS. 23 and 24. FIGS. 23 and 24 are explanatory diagrams for describing exemplary information processing according to Modification 1 of the present embodiment. FIGS. 23 and 24 illustrate temporally sequential data of the HRV indexes of target users (in FIGS. 23 and 24, Person A and Person B) during the brainstorming and predicted in the future. Specifically, similarly to FIGS. 21 and 23, FIGS. 23 and 24 each illustrate the temporally sequential data of the HRV index of Person A at the upper part and illustrate the temporally sequential data of the HRV index of Person B at the lower part.

The following first describes a case in which Person A and Person B can be estimated to be in concentration in Duration S and Duration U in brainstorming, and in addition, Person A and Person B can be expected to be in concentration in Duration T and Duration V in the future as illustrated in FIG. 23. In such a case, the server 30 determines that concentration of the target users can be maintained in the future, and selects to perform notification that suggests continuation of the brainstorming to the facilitator.

Subsequently, the case illustrated in FIG. 24 is described. In FIG. 24, Person A can be estimated to be in concentration in Duration AA in brainstorming, and in addition, is expected to be in concentration in Duration AB in the future. However, Person B can be estimated to be in concentration in the first duration AC in the brainstorming but can be estimated not to be in concentration or to be sleepy in the next duration AD. In addition, Person B is predicted not to be in concentration or to be sleepy in Duration AE in the future based on the tendency in Duration AC and Duration AD. In such a case, since the concentration degree of Person B participating in the brainstorming is expected to decrease in the future, the server 30 predicts the concentration degree of any other participating user to decrease in the future. In addition, the server 30 determines based on the expectation that it is difficult to effectively proceed the brainstorming in the future, and selects to perform notification that suggest cancellation or interruption of the brainstorming to the facilitator.

As described above, in the present modification, since the concentration degree of the target user in the future can be predicted, for example, whether to continue the above-described brainstorming can be selected based on the prediction. Thus, according to the present modification, whether to continue the brainstorming can be objectively determined, and thus the facilitator can appropriately proceed the brainstorming and derive the maximum effect of the brainstorming.

In the present modification, the server 30 can predict the continuation time of concentration of the target user in the future, and thus may specifically notify based on the predicted continuation time, to the facilitator, the continuation time of the brainstorming or a time at which the brainstorming is recommended to end. In addition, in the present modification, the server 30 can predict the tendency of concentration of the target user in the future, for example, how long a break is needed to recover the concentration of the target user, and thus may specifically suggest, to the facilitator, a break time with which the concentration is expected to recover.

### <4.3 Modification 2>

As described above, the success of brainstorming often depends on the configuration of members, but it has been difficult to verify a synergy effect due to the member configuration, in other words, compatibility. For example, when one of members participating in brainstorming leads the brainstorming and has a successful outcome or the like, it is likely that the brainstorming is determined to be successful because of the successful outcome. However, when any other member does not actively participate in the brainstorming, the synergy effect is not obtained in reality and the brainstorming is hardly successful.

Thus, in Modification 2 of the present embodiment, the emotion of a group performing brainstorming is estimated by using the above-described information processing system 1, and the existence of the synergy effect, in other words, the member configuration (combination or compatibility) is objectively evaluated based on a result of the estimation. Then, in the present modification, members in the next brainstorming are suggested based on the evaluation of the member configuration. The suggestion can be performed by, for example, causing a PC (terminal 50) used by a facilitator of the next brainstorming to display, on a screen, the names of the suggested members.

For example, as illustrated in FIG. 21 described above, when each target user (Person A and Person B) is estimated to be in concentration in brainstorming, the server 30 evaluates that combination of Person A and Person B participating in the brainstorming has appropriate compatibility. In such a case, the server 30 suggests combining, in the next brainstorming, Person A and Person B having compatibility evaluated to be appropriate.

As illustrated in FIG. 22 described above, when Person A is estimated to be in concentration but Person B is estimated not to be in concentration in brainstorming, the server 30 evaluates that Person A and Person B participating in the brainstorming have inappropriate compatibility. In such a case, the server 30 suggests not to combine, in the next brainstorming, Person A and Person B having compatibility evaluated to be inappropriate.

As described above, according to the present modification, the emotion of a group performing brainstorming is estimated, and combination is objectively evaluated based on a result of the estimation. In addition, according to the present modification, combination having compatibility estimated to be appropriate in brainstorming can be suggested based on the combination evaluation obtained in this manner. Thus, according to the present modification, the brainstorming can be more effectively performed with reference to such suggestion.

In addition, in the present modification, sensing data obtained by the sound sensor (not illustrated) included in the sensor unit 120 may be used. For example, when the existence of a user decreasing the concentration degree of any other target user through statements in brainstorming has been detected based on the sensing data obtained by the sound sensor, the server 30 suggests removing the user in the next brainstorming. In addition, the server 30 may store, for a user determined to have a high tendency to provide a statement that decreases the concentration degree of any other target user for a plurality of times of brainstorming, information of the tendency in a database or the like.

In the present modification, combination may be evaluated with reference to not only the emotion of each target user but also, for example, evaluation of a deliverable of brainstorming. In this case, the server 30 evaluates the combination in the brainstorming to be appropriate when the concentration degree of each participating target user is high and the deliverable evaluation is high. When the deliverable evaluation is high but the concentration degree of any participating target user is low, the server 30 evaluates the combination in the brainstorming to be inappropriate.

The brainstorming deliverable evaluation may be performed based on indexes such as the number of ideas and the number of statements, or may be obtained through evaluation of a deliverable (report) or the like by a person not participating in the brainstorming or the like. Alternatively, any user participating in the brainstorming may be asked for a questionnaire after the brainstorming ends, and the brainstorming deliverable evaluation may be performed based on the results of the questionnaire.

### <4.4 Modification 3>

When brainstorming is being performed with the same members, freshness is gradually lost and the brainstorming does not become active in some cases. Thus, in such a case, to conduct active brainstorming and also to obtain new knowledge, it is thought to be effective to have a new member in the next brainstorming. Typically, in brainstorming, a better outcome is obtained when participating members are different from each other in background or the like. However, when brainstorming is performed by members having different concentration and excitation tendencies, discussion does not proceed well due to the tendency difference, and the brainstorming does not become active in some cases. In other words, brainstorming is likely to become active when members having similar concentration and excitation tendencies are combined.

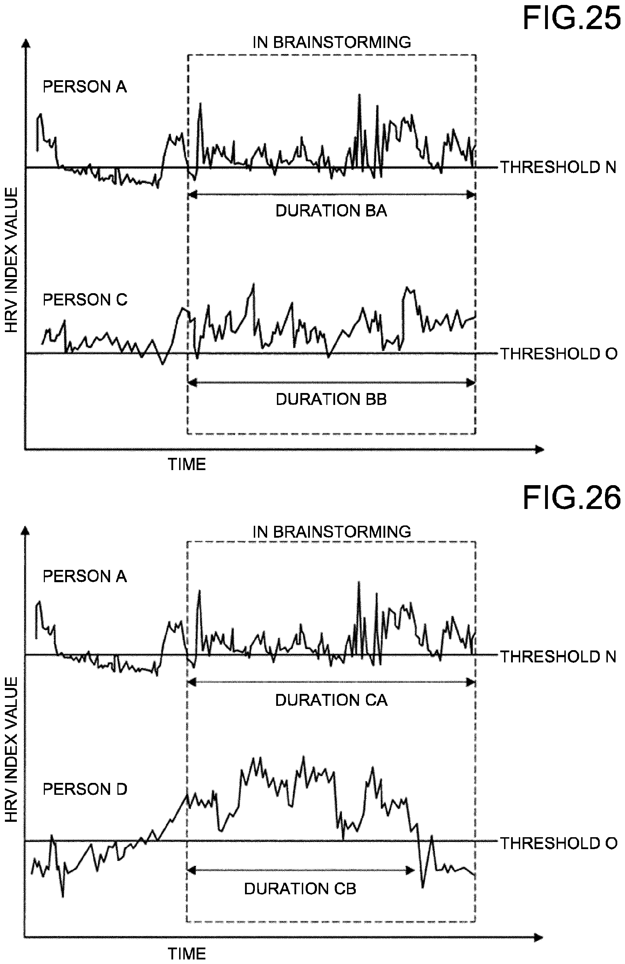

Thus, in the present modification, a target user having a similar tendency is extracted based on the tendency of the emotion of each target user in past brainstorming, and the extracted target user is suggested as a new member or a replacement member (candidate) in the next brainstorming. The following describes such a modification with reference to FIGS. 25 and 26. FIGS. 25 and 26 are explanatory diagrams for describing exemplary information processing according to Modification 3 of the present embodiment. Similarly to FIGS. 21 and 22, FIGS. 25 and 26 illustrate temporally sequential data of the HRV indexes of target users (in FIGS. 25 and 26, Person A, Person C, and Person D) in brainstorming.

For example, in the case illustrated in FIG. 25, Person A can be estimated to be in concentration in Duration BA in brainstorming because the HRV index is higher than Threshold N. Similarly, Person C can be estimated to be in concentration in Duration BB in the brainstorming because the HRV index is higher than Threshold W. In such a case, the server 30 determines that Person A and Person C have similar concentration tendencies because they are in concentration with the same tendency in the same brainstorming.

In the case illustrated in FIG. 26, Person A can be estimated to be in concentration in Duration CA in brainstorming because the HRV index is higher than Threshold N. Similarly, Person D can be estimated to be in concentration in Duration CB in the brainstorming, which is shorter than Duration CA, because the HRV index is higher than Threshold X. In such a case, the server 30 determines that Person A and Person D have similar concentration tendencies because they are in concentration with substantially the same tendency in the same brainstorming.

Then, based on the concentration tendencies as illustrated in FIGS. 25 and 26, the server 30 suggests, as a new member in the next brainstorming, Person D having a tendency similar to that of Person A in place of Person C having a tendency similar to that of Person A. The suggestion can be performed by, for example, causing a PC (terminal 50) used by a facilitator of the next brainstorming to display the name of the suggested new member on a screen.

As described above, in the present modification, a target user having a similar tendency is extracted, and the extracted target user is objectively suggested as a new member in the next brainstorming. Thus, according to the present modification, the next brainstorming can be made active through participation of the new member based on such suggestion.

### <4.5 Modification 4>

The above-described present embodiment and modifications are used in brainstorming but are not limited to use in brainstorming and may be used in a council and a fair. The council and the fair are typically performed to review and evaluate matters by a plurality of members, and each member is required to be calm and fair and objectively participating in concentration.

Thus, in the present modification, the emotion of a group carrying out the council or the like is estimated by using the above-described information processing system 1, whether each member is calm and fair and objectively participating in concentration is evaluated based on a result of the estimation, and a result of the evaluation is presented to a facilitator of the council or the like. Specifically, when there are opinions opposing to each other in the council or the like, some members become excited and critical in some cases because of the bad compatibility of opposing members. In such a case, calm thought and determination can be stopped by excitation or the like, and thus it is difficult to appropriately proceed the council. Thus, in the present modification, whether a member in excitation or the like exists is estimated by using the above-described information processing system 1, and the existence of such a member is notified to the facilitator of the council or the like. Then, in such a case, the facilitator can select an appropriate measure such as a break based on the notification so that the member becomes calm.

When a council or the like is performed while some members are not in concentration and sleepiness is high, the council or the like cannot be thought to be performed fair in some cases because interest and observation on a review target by the members are insufficient. Thus, in the present modification, the emotion of a group carrying out in the council or the like is estimated by using the above-described information processing system 1, whether each member is in concentration is evaluated based on a result of the estimation, and a result of the evaluation is presented to a facilitator of the council or the like. More specifically, when the concentration degree of the group carrying out the council or the like is estimated to be insufficient by the above-described information processing system 1 and the insufficiency of the concentration degree is notified, the facilitator can suggest a break or an interruption of the council or the like based on the notification.

As described above, in the present modification, the emotion of a group can be objectively estimated, and a result of the estimation can be notified to a facilitator or the like, and thus, the facilitator can appropriately proceed a council or the like in which each member is required to be calm and fair and objectively participating in concentration.

### <<5. Third embodiment>>

In the above-described embodiments, the emotion of a target user is estimated based on sensing data obtained by mainly various biosensors, and output is performed in accordance with a result of the estimation, but the embodiment of the present disclosure is not limited thereto. For example, in the embodiment of the present disclosure, the state (for example, use status) of a group including a plurality of target users may be estimated, and output may be performed in accordance with a result of the estimation. Thus, in a third embodiment described below, information related to the use statuses of a plurality of users is acquired, the states of the users are estimated based on the acquired information, and a facility or the like around the users is controlled based on a result of the estimation. Thus, according to the present embodiment describes below, needs that could occur in estimated states of a plurality of users can be dealt with.

More specifically, the present embodiment describes application to control the air conditioning facility 602 (refer to FIG. 28) (the terminal 50) in a cinema. In many conventional cases, in a cinema or the like, the air conditioning facility 602 or the like cannot be controlled in accordance with the use status of the audience (user) or the like. Thus, in the present embodiment, the server 30 estimates the state of the audience by using the above-described information processing system 1 and controls the air conditioning facility 602 in accordance with a result of the estimation, thereby providing a comfortable space to the audience.

In the present embodiment, the configurations of the information processing system 1, the wearable device 10, the server 30, and the terminal 50 are common to those in the first embodiment, and the description of these configurations in the first embodiment can be referred to. Thus, in the following, description of detailed configurations of the information processing system 1, the wearable device 10, the server 30, and the terminal 50 will be omitted. In the present embodiment, the wearable device 10 may not be included in the information processing system 1.

### <5.1 Information processing method>

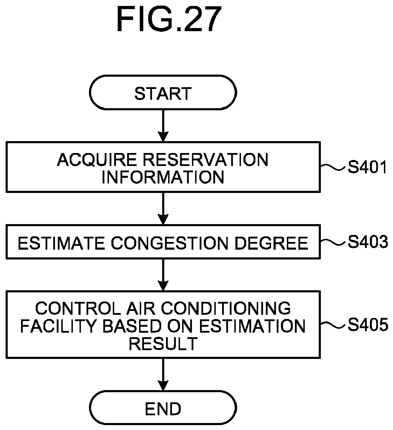

The following describes the information processing method according to the present embodiment with reference to FIGS. 27 and 28. FIG. 27 is a flowchart for describing an exemplary information processing method according to the present embodiment. FIG. 28 is an explanatory diagram for describing exemplary information processing according to the present embodiment, and specifically schematically illustrates an inside 600 of a cinema or the like, and the air conditioning facility 602 configured to adjust air conditioning of the inside.

As illustrated in FIG. 27, the information processing method according to the present embodiment includes a plurality of steps S401 to S405. The following describes details of each step included in the information processing method according to the present embodiment.

### (Step S401)

The server 30 acquires, as sensing data, reservation information input from each user through the terminal 50. Specifically, the user inputs, as seat reservation, information such as a cinema at which viewing is desired, a movie to be screened, and the number of seats.

### (Step S403)

The server 30 estimates a congestion degree at the cinema from the acquired reservation information.

### (Step S405)

The server 30 controls the air conditioning facility 602 of the inside 600 of the cinema in accordance with a result of the estimation. Specifically, when congestion is estimated at step S405 described above, the server 30 controls the air conditioning facility 602 to cool the inside 600. When congestion is not estimated at step S405 described above, the server 30 controls the air conditioning facility 602 not to cool the inside 600 too much.

When estimating the congestion degree, the server 30 may forecast the male-female ratio of the audience or the like with the screening date and time and the screening content taken into consideration. More specifically, for example, the number of family viewers tends to be large or the number of lone viewers tends to be large in accordance with the screening time and the screening content. Thus, the server 30 can predict the number and male-female ratio of audience in advance by referring to information such as the screening date and time. Since appropriate temperature differs between the male and the female, the server 30 controls the air conditioning facility 602 in accordance with the predicted male-female ratio. The server 30 may determine the male-female ratio of the audience in advance by using seat reservation. In addition, the server 30 may improve the accuracy of the estimation by referring to information at any other cinema.

In addition, in the present embodiment, the congestion degree of the cinema is estimated based on the reservation information acquired in advance, but the congestion degree of the cinema may be also estimated based on, for example, sensing data obtained by a pressure sensor (not illustrated) installed at each seat provided to the inside 600. Alternatively, for example, in the present embodiment, the congestion degree of the cinema may be estimated by analyzing an image capturing image as sensing data obtained by an image capturing apparatus (not illustrated) provided to the inside 600.

In the present embodiment, how the audience viewing a movie feels the temperature of the inside 600 may be estimated based on sensing data obtained by, for example, a sweating sensor (not illustrated) included in the sensor unit 120 of the wearable device 10 mounted on the audience.

As described above, in the present embodiment, the congestion degree of the audience of a cinema is estimated, and the air conditioning facility 602 or the like of the inside 600 is controlled in accordance with a result of the estimation. Thus, in the present embodiment, not only the state of the audience (user) is estimated, but also the needs of the audience are specified in accordance with the estimated state, and the air conditioning facility 602 is controlled to deal with the specified needs. Thus, according to the present embodiment, the audience can create a situation preferable for the audience without taking any action in particular.

The present embodiment is not limited to control of the air conditioning facility 602 of the inside 600, but for example, the server 30 may transmit a notification that suggests clothes and belongings to the audience in advance by mail or the like in accordance with an estimation result. More specifically, in the present embodiment, for example, the air conditioning facility 602 is sometimes controlled to perform stronger cooling when the male audience ratio is high because the male highly tends to feel warm. Thus, in such a case, the server 30 sends a mail that suggests bringing a cloth such as a stall or a cardigan with which temperature adjustment can be performed to the female audience who highly tends to feel cold, in advance to, for example, a smartphone (terminal 50) used by the audience. In addition, in such a case, the server 30 may perform, to a person in charge of screening at the cinema, notification that suggests, for example, preparation of enough numbers of blankets, hot drinks, and snacks to be provided in the inside 600.

In the present embodiment, after the movie screening ends, the audience may be asked to answer a questionnaire about the air conditioning of the inside 600 or the like to acquire evaluation, and the evaluation may be reflected on control of the air conditioning facility 602 at the next screening. In this manner, it is possible to provide a situation more comfortable for the audience.

The above description is made on use at the inside 600 of the cinema, but the present embodiment can be used at places other than a cinema, for example, at public places such as a museum, a gallery, a hotel, and a restaurant where various kinds of people gather.

The present embodiment can be used for the air conditioning facility 602 of a vehicle such as a bus or a taxi. In this case, the server 30 acquires the position of the vehicle and time as sensing data. Specifically, near an office block, a school, or the like, the occupancy of the vehicle is estimated to be high in time slots corresponding to commuting times in morning and evening on a weekday. In downtown including a large-scale commercial facility, the occupancy of the vehicle is estimated to be high on a holiday. Thus, the server 30 estimates the occupancy at the vehicle position and the date and time acquired as sensing data based on the tendencies as described above, and controls the air conditioning facility 602 of the vehicle in accordance with a result of the estimation. In addition, the server 30 may acquire the temperature and expected occupancy of the vehicle, and may provide the temperature and occupancy of the vehicle to any user intended to use the vehicle.

In a case as described above, the server 30 may acquire, as sensing data, reservation information of a bus or a taxi, which is input from each user through the terminal 50. In this case, the server 30 may acquire air conditioning preference (for cool air or warm air, for example) of each user together with the reservation information, and may utilize the acquired information in control of the air conditioning facility 602 of the vehicle.

### <5.2 Modification 1>

In the third embodiment described above, the congestion degree at a cinema is estimated, but the embodiment is not limited thereto, and a facility use frequency at a public facility may be estimated, and for example, the number of times of cleaning or the like may be notified to a facility administrator in accordance with a result of the estimation. The following describes such an example as Modification 1 according to the third embodiment.

For example, typically, a facility such as a toilet of a public facility is not cleaned in accordance with the use frequency thereof but is periodically cleaned at determined date and time. In this manner, the facility can be constantly maintained clean, but it is difficult to reduce the cost of cleaning or the like. Thus, the information processing system 1 according to the present embodiment can be used to estimate the use frequency and suggest an appropriate cleaning timing or the like to a facility administrator in accordance with a result of the estimation, thereby maintaining the facility clean and reducing the cost of cleaning. In addition, in the present modification, the server 30 may suggest the number of persons, an equipment, and a solvent for the cleaning in accordance with the estimation result. The suggestion can be performed by, for example, causing a PC (terminal 50) used by the facility administrator to display the cleaning date and time, the number of persons, and the like on a screen.

Specifically, the server 30 estimates the use frequency of the facility by target users based on sensing data obtained by, for example, the positioning sensor (not illustrated) included in the sensor unit 120 of the wearable device 10 mounted on each target user. Specifically, the server 30 estimates the use frequency by determining, based on the sensing data, the number of target users staying in the facility for a predetermined duration. When the facility is a toilet, the server 30 may estimate the use frequency by acquiring the number of times of opening and closing of a stall door based on sensing data obtained by an opening sensor (not illustrated) installed on the stall door. Similarly, the server 30 may estimate the use frequency based on, for example, the number of times of water flush or the number of times that illumination is turned on. Then, the server 30 suggests the timing of cleaning or the like to the facility administrator in accordance with the estimated use frequency, thereby maintaining the facility clean and reducing the cost of cleaning. In addition, when the facility is a toilet and the number of times of opening and closing of a door is large but the number of times of water flush is extremely small, the server 30 estimates that there is a person who has tried to use the toilet but stopped using because the toilet is extremely dirty, and suggests urgent cleaning to the facility administrator.

As described above, according to the present modification, the use frequency of a facility is estimated and the timing of cleaning or the like is suggested to a facility administrator in accordance with a result of the estimation, thereby maintaining the facility clean and reducing the cost of cleaning. Specifically, in the present modification, not only the state of each user can be estimated, but also needs of the user can be specified in accordance with the estimated state, and the facility administrator can be suggested to deal with the specified needs.

### <5.3 Modification 2>

The above-described estimation of the congestion degree and use frequency of a public facility by the server 30 may be used to estimate, for example, the use status of a locker installed at the public facility and notify the use status to a user of the public facility. More specifically, in the present modification, application is made to estimate the use status of a locker installed at, for example, a railway station, an airport, a theater, a public bath.

Normally, the number of users of the public facility and the number of locker users do not necessarily match each other in many cases. Thus, in the present modification, similarly to the above-described modification, the server 30 estimates the congestion degree of the public facility, and also acquires a current locker use status from, for example, an opening sensor (not illustrated) provided to each locker. Then, the server 30 predicts the use status of the locker in the future based on the estimated congestion degree and the acquired use status. In addition, based on a result of the prediction, the server 30 displays, to any user of the public facility who potentially uses the locker, the existence of any available locker, a time slot in which the locker is available, and the like on, for example, a display device (terminal 50) (not illustrated) installed at the public facility. Based on the display, the user can consider use of the locker or shift a time in which the locker is to be used. In addition, the server 30 may notify the prediction result to an administrator of the locker, and in this case, may suggest, to the administrator, change of the number of installed lockers in accordance with the use status, and change of the usage fee of the locker for adjusting the use status.

The present modification describes use for a locker but is not limited to such use, and may be used at for example, an amusement park. In this case, the server 30 can estimate the position of each user and a congestion status based on sensing data obtained by, for example, the positioning sensor (not illustrated) included in the sensor unit 120 of the wearable device 10 mounted on the user (target user). Then, when a position at which the congestion degree is significantly high is detected, the server 30 presumes that discomfort of users is highly likely to increase due to congestion, and suggests, to a facility administrator, for example, deployment of staffs and casts who can make the users happy.

### <<6. Summary>>

As described above, according to the embodiment of the present disclosure, it is possible to provide the information processing system 1 capable of not only estimating the state of a target user but also dealing with needs of the target user that could occur in the estimated state. As a result, according to the present embodiment, the target user can create a situation preferable for the target user without taking any action in particular.

### <<7. Hardware configuration>>

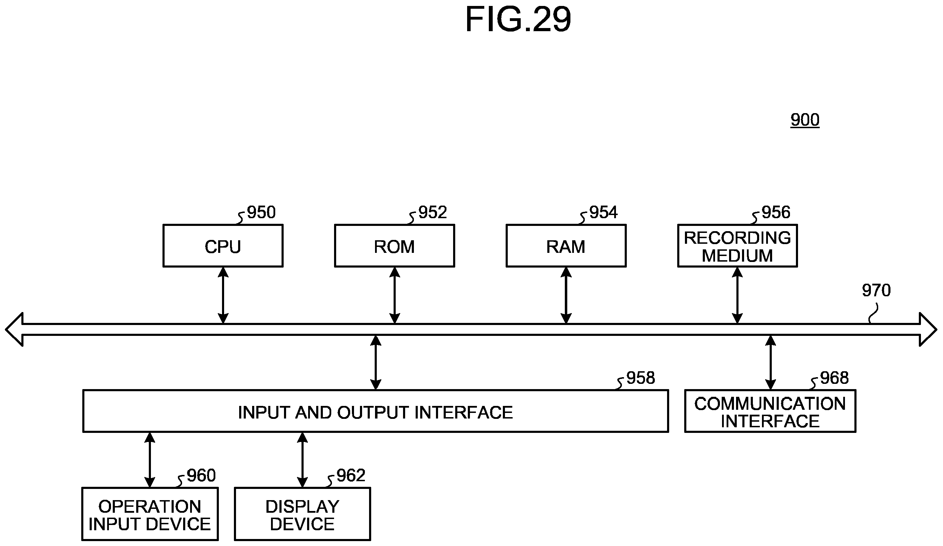

FIG. 29 is an explanatory diagram illustrating an exemplary hardware configuration of an information processing device 900 according to the present embodiment. In FIG. 29, the information processing device 900 indicates an exemplary hardware configuration of the above-described server 30.

The information processing device 900 includes, for example, a CPU 950, a ROM 952, a RAM 954, a recording medium 956, an input and output interface 958, and an operation input device 960. In addition, the information processing device 900 includes a display device 962 and a communication interface 968. In the information processing device 900, the components are connected with each other through, for example, a bus 970 as a data transmission path.

### (CPU 950)

The CPU 950 includes, for example, one processor or two or more processes each achieved by a calculation circuit such as a CPU, and various kinds of processing circuits, and functions as a control unit configured to control the entire information processing device 900. Specifically, the CPU 950 functions as, for example, the above-described control unit 330 in the information processing device 900.

### (ROM 952 and RAM 954)

The ROM 952 stores computer programs and control data such as calculation parameters, which are used by the CPU 950. The RAM 954 temporarily stores, for example, computer programs to be executed by the CPU 950.

### (Recording medium 956)

The recording medium 956 functions as the above-described storage unit 360, and stores various kinds of data such as data related to the information processing method according to the present embodiment and various applications. Examples of the recording medium 956 include a magnetic recording medium such as a hard disk, and a nonvolatile memory such as a flash memory. The recording medium 956 may be detachable from the information processing device 900.

### (Input and output interface 958, operation input device 960, and display device 962)

The input and output interface 958 connects, for example, the operation input device 960 and the display device 962. Examples of the input and output interface 958 include a universal serial bus (USB) terminal, a digital visual interface (DVI) terminal, a High-Definition Multimedia Interface (HDMI) (registered trademark) terminal, and various kinds of processing circuits.

The operation input device 960 functions as, for example, the above-described input unit 300, and is connected with the input and output interface 958 inside the information processing device 900.

The display device 962 functions as, for example, the above-described output unit 310, and is provided on the information processing device 900 and connected with the input and output interface 958 inside the information processing device 900. Examples of the display device 962 include a liquid crystal display and an organic electro-luminescence display (organic EL display).

The input and output interface 958 can be connected with external devices such as an operation input device (for example, a keyboard or a mouse) and a display device outside the information processing device 900.

The input and output interface 958 may be also connected with a drive (not illustrated). The drive is a reader-writer for a removable recording medium such as a magnetic disk, an optical disk, or a semiconductor memory, and is built in or externally connected with the information processing device 900. The drive reads information recorded in a removable recording medium mounted thereon, and outputs the read information to the RAM 954. In addition, the drive can write a record to a removable recording medium mounted thereon.

### (Communication interface 968)

The communication interface 968 functions as the above-described communication unit 350 for performing communication with an external device such as the server 30 in a wireless or wired manner through, for example, the network 70 (or directly). Examples of the communication interface 968 include a communication antenna and a radio frequency (RF) circuit (wireless communication), an IEEE802.15.1 port and a transmission and reception circuit (wireless communication), an IEEE802.11 port and a transmission and reception circuit (wireless communication), and a local area network (LAN) terminal and a transmission and reception circuit (wired communication).

The exemplary hardware configuration of the information processing device 900 is described above. However, the hardware configuration of the information processing device 900 is not limited to the configuration illustrated in FIG. 29. Specifically, each above-described component may be configured by using a general-purpose member or may be configured by hardware specialized for the function of the component. Such a configuration is changed as appropriate in accordance with a technology level when the present embodiment is performed.

The information processing device 900 according to the present embodiment may be applied to a system including a plurality of devices based on an assumption of connection to a network (or communication between the devices) as in, for example, cloud computing. In other words, the above-described information processing device 900 according to the present embodiment may be achieved as, for example, the information processing system 1 in which processing of the information processing method according to the present embodiment is performed by a plurality of devices.

### <<8. Supplement>>

The above-described embodiment of the present disclosure includes, for example, a computer program configured to cause a computer to function as the information processing device according to the present embodiment, and a non-transitory physical medium in which the computer program is recorded. The computer program may be distributed through a communication line (including wireless communication) such as the Internet.

Steps in the processing of each above-described embodiment do not necessarily need to be processed in the stated order. For example, the steps may be processed in an order changed as appropriate. The steps may be processed partially in parallel or individually instead of being processed in a temporally sequential manner. In addition, the processing method of each step does not necessarily need to be processed by the stated method but may be processed, for example, by another functional component by another method.

Preferable embodiments of the present disclosure are described above in detail with reference to the accompanying drawings, but the technical scope of the present disclosure is not limited to such examples. Various changes and modifications could be thought of by a person having typical knowledge in the technical field of the present disclosure within the range of the technical idea written in the claims, and it should be understood that these changes and modifications belong to the technical scope of the present disclosure.

Effects stated in the present specification are explanatory or exemplary but not restrictive. Thus, the technology according to the present disclosure achieves, together with or in place of the above-described effects, any other effect that is obvious to the skilled person in the art from description of the present specification.

Configurations as described below belong to the technical scope of the present disclosure.
(1) An information processing device comprising:
   an estimation unit configured to estimate states of one or a plurality of target users based on sensing data; and
   an output control unit configured to perform output corresponding to an estimation result.
(2) The information processing device according to (1), wherein the estimation unit estimates emotion of each target user based on sensing data obtained by a biosensor mounted on the target user.
(3) The information processing device according to (2), wherein the estimation unit estimates emotion of each target user after the sensing data is acquired.
(4) The information processing device according to (2) or (3), further comprising a learning unit configured to learn emotional tendency of the target user by using the sensing data acquired in the past.
(5) The information processing device according to (4), further comprising an evaluation acquisition unit configured to acquire evaluation of content of the output by the target user, wherein the learning unit learns the emotional tendency of the target user based on the evaluation.
(6) The information processing device according to any one of (2) to (5), wherein the output control unit performs, as the output, control of a device used by the target user or a device installed near the target user.
(7) The information processing device according to any one of (2) to (5), wherein the output control unit performs, as the output, control of a device used by a user included in a group to which the target user belongs.
(8) The information processing device according to (6) or (7), wherein the output control unit controls the device to display a recommended action in accordance with the estimation result.
(9) The information processing device according to (2), wherein the estimation unit estimates emotion of a group of a plurality of the target users.
(10) The information processing device according to (9), wherein the output control unit performs, as the output, control of a device used by a user included in the group to display a recommended action in accordance with the estimation result.
(11) The information processing device according to (9) or (10), wherein the output control unit performs combination evaluation of combinations of the target users included in the group based on the estimation result.
(12) The information processing device according to (11), further comprising an evaluation acquisition unit configured to acquire deliverable evaluation of a deliverable of the group, wherein the output control unit performs the combination evaluation based on the deliverable evaluation.
(13) The information processing device according to (12), wherein the output control unit outputs candidates of the target users for next combination based on the combination evaluation.
(14) The information processing device according to any one of (2) to (13), wherein the sensing data is acquired by a beat sensor directly mounted on part of the body of the target user and configured to detect heart rate or pulse rate.
(15) The information processing device according to any one of (2) to (13), wherein the sensing data is acquired from a sweating sensor directly mounted on part of the body of the target user and configured to detect sweating.
(16) The information processing device according to any one of (2) to (13), wherein the sensing data is acquired from a sensor directly mounted on part of the body of the target user and configured to detect at least one of blood pressure, brain wave, breathing, myopotential, and skin temperature.
(17) The information processing device according to (1), wherein the estimation unit estimates the state of a group of a plurality of the target users based on sensing data input by the target users.
(18) The information processing device according to (17), wherein the output control unit performs, as the output, control of a device installed around the group.
(19) An information processing method comprising:
   estimating states of one or a plurality of target users based on sensing data; and
   performing output corresponding to an estimation result. (20)

A computer program configured to cause a computer to execute:
a function to estimate states of one or a plurality of target users based on sensing data; and
a function to perform output corresponding to an estimation result.

### Reference Signs List

- 1: information processing system
- 10: wearable device
- 12: band unit
- 14: control unit
- 30: server
- 50: terminal
- 70: network
- 100, 300, 500: input unit
- 110, 310, 510: output unit
- 120: sensor unit
- 122: PPG sensor unit
- 124: motion sensor unit
- 130, 330, 530: control unit
- 150, 350, 550: communication unit
- 160, 360, 560: storage unit
- 200: measurement site
- 202: blood vessel
- 332: sensing data acquisition unit
- 334: processing unit
- 336: output control unit
- 338: evaluation acquisition unit
- 340: estimation unit
- 342: learning unit
- 362: DB
- 600: inside
- 602: air conditioning facility
- 700: display lamp
- 800, 802, 804, 806: screen
- 850: marker
- 860: comment
- 900: information processing device
- 950: CPU
- 952: ROM
- 954: RAM
- 956: recording medium
- 958: input and output interface
- 960: operation input device
- 962: display device
- 968: communication interface
- 970: bus

## Claims

1. An information processing device comprising:
an estimation unit configured to estimate states of one or a plurality of target users based on sensing data; and
an output control unit configured to perform output corresponding to an estimation result.

2. The information processing device according to claim 1, wherein the estimation unit estimates emotion of each target user based on sensing data obtained by a biosensor mounted on the target user.

3. The information processing device according to claim 2, wherein the estimation unit estimates emotion of each target user after the sensing data is acquired.

4. The information processing device according to claim 2, further comprising a learning unit configured to learn emotional tendency of the target user by using the sensing data acquired in the past.

5. The information processing device according to claim 4, further comprising an evaluation acquisition unit configured to acquire evaluation of content of the output by the target user, wherein the learning unit learns the emotional tendency of the target user based on the evaluation.

6. The information processing device according to claim 2, wherein the output control unit performs, as the output, control of a device used by the target user or a device installed near the target user.

7. The information processing device according to claim 2, wherein the output control unit performs, as the output, control of a device used by a user included in a group to which the target user belongs.

8. The information processing device according to claim 6, wherein the output control unit controls the device to display a recommended action in accordance with the estimation result.

9. The information processing device according to claim 2, wherein the estimation unit estimates emotion of a group of a plurality of the target users.

10. The information processing device according to claim 9, wherein the output control unit performs, as the output, control of a device used by a user included in the group to display a recommended action in accordance with the estimation result.

11. The information processing device according to claim 9, wherein the output control unit performs combination evaluation of combinations of the target users included in the group based on the estimation result.

12. The information processing device according to claim 11, further comprising an evaluation acquisition unit configured to acquire deliverable evaluation of a deliverable of the group, wherein the output control unit performs the combination evaluation based on the deliverable evaluation.

13. The information processing device according to claim 12, wherein the output control unit outputs candidates of the target users for next combination based on the combination evaluation.

14. The information processing device according to claim 2, wherein the sensing data is acquired by a beat sensor directly mounted on part of the body of the target user and configured to detect heart rate or pulse rate.

15. The information processing device according to claim 2, wherein the sensing data is acquired from a sweating sensor directly mounted on part of the body of the target user and configured to detect sweating.

16. The information processing device according to claim 2, wherein the sensing data is acquired from a sensor directly mounted on part of the body of the target user and configured to detect at least one of blood pressure, brain wave, breathing, myopotential, and skin temperature.

17. The information processing device according to claim 1, wherein the estimation unit estimates the state of a group of a plurality of the target users based on sensing data input by the target users.

18. The information processing device according to claim 17, wherein the output control unit performs, as the output, control of a device installed around the group.

19. An information processing method comprising:
estimating states of one or a plurality of target users based on sensing data; and
performing output corresponding to an estimation result.

20. A computer program configured to cause a computer to execute:
a function to estimate states of one or a plurality of target users based on sensing data; and
a function to perform output corresponding to an estimation result.
